# EUROPÄISCHE PATENTANMELDUNG

(11) **EP 3 919 122 A2**
(43) Veröffentlichungstag der Anmeldung: **08.12.2021**
(21) Anmeldenummer: 21177470.8
(22) Anmeldetag: 02.06.2021
(51) Int. Cl.: A61N 5/06

(54) **LICHTTHERAPIEKAMMER MIT WENIGSTENS ZWEI STEUER- UND/ODER ÜBERWACHUNGSEINHEITEN UND VERFAHREN ZUR ÜBERWACHUNG EINER LICHTTHERAPIEKAMMER**

(30) Priorität: 02.06.2020 DE 102020114666
(71) Anmelder: RP Medics GmbH, 63110 Rodgau (DE)
(72) Erfinder: Pasedag, Roland, 63110 Rodgau (DE); Pasedag, Reinald, 63110 Rodgau (DE)
(74) Vertreter: Cremer & Cremer

(57) **Zusammenfassung**

Eine Lichttherapiekammer, beispielsweise als verschließbare Zelle, die mindestens das Eintreten und Aufhalten eines Menschen in ihr möglich macht, ist mit einer ersten elektronischen Steuerungs- und Überwachungseinheit ausgestattet. Die Steuerungs- und Überwachungseinheit steht mit Lichtquellen der Lichttherapiekammer in elektronischer Verbindung. So kann die Steuerungs- und Überwachungseinheit zum Ein- und Ausschalten und zur Funktionsüberwachung der Lichttherapiekammer genutzt werden. Eine zweite Steuereinheit kann über eine Internetverbindung die von der ersten Steuerungs- und Überwachungseinheit erfassten (Mess-)Daten empfangen und speichern. Die zweite Steuereinheit kann außerdem in die Funktion der ersten Steuerungs- und Überwachungseinheit, also in den Betriebszustand der Lichttherapiekammer eingreifen. So kann aus der Ferne festgestellt werden, dass die Lichttherapiekammer in einem unerwünschten, die Sicherheit des Nutzers möglicherweise gefährdenden Betriebszustand ist.

## Beschreibung

Die vorliegende Erfindung behandelt eine Lichttherapiekammer mit einer elektronischen Steuerungs- und Überwachungseinheit, über die mehrere Lichtquellen angesteuert werden können.

Des Weiteren behandelt die vorliegende Erfindung ein Verfahren zur Überwachung einer Lichttherapiekammer mithilfe einer Überwachungseinheit, die die Funktionstüchtigkeit von Leuchtmitteln der Lichttherapiekammer überwachen kann.

Mit anderen Worten, die vorliegende Erfindung behandelt eine Lichttherapiekammer nach dem Oberbegriff von Anspruch 1 sowie ein Verfahren zur Überwachung einer entsprechenden Lichttherapiekammer nach dem Oberbegriff von Anspruch 14.

### Technisches Gebiet

Aus zahlreichen medizinischen Untersuchungen ist bekannt, dass mit Hilfe von Licht ausgewählter Wellenlängen positive Effekte bei äußerlich bestrahlten Personen sowie bei einer Effusion von Körperflüssigkeiten wie Blut oder Plasma (siehe z. B. "A new proof of concept in bacterial reduction" von Michelle Maclean et al. in Journal of Blood Transfusion, Volume 2016) herbeigeführt werden kann. Sowohl aus der Fachliteratur als auch aus der Patentliteratur lassen sich Hinweise entnehmen, dass nicht nur ultraviolettes Licht und infrarotes Licht positive Effekte bei dem Bestrahlen von Lebewesen, wie Menschen oder Tieren, sondern auch einzelne Frequenzen und Frequenzbänder im optischen bzw. sichtbaren Wellenspektrum ebenfalls positive Effekte hervorrufen können.

Das für Menschen sichtbare Wellenspektrum wird üblicherweise in einem Wellenlängenbereich zwischen 400 nm und einem Wellenlängenbereich unterhalb von 750 nm angesiedelt. Die Breite und die exakte Lage des Frequenzbandes, also welche Wellenlängen tatsächlich sichtbarem Licht entsprechen, bzw. die Wellenlängengrenzwerte des Wellenlängenbandes variieren etwas von Mensch zu Mensch. Auch gilt bei manchen Tieren ein etwas anderes Wellenband als sichtbares Lichtwellenband, d. h. in dem jene Tiere Licht optisch wahrnehmen können (z. B. ist das Wellenlängenband bei Honigbienen um ca. 150 nm in Richtung auf das kurzwelligere UV-Licht verschoben). Generell kann aber gesagt werden, dass als optisch sichtbares Wellenlängenband ein Wellenlängenband angesehen werden kann, das zwar in den Randbereichen bzw. bezüglich seiner Grenzwerte um einige nm variieren kann, das jedoch eine Bandbreite von ca. 350 nm überstreicht. Dieser Wellenlängenbereich wird gemeinhin als sichtbarer Wellenlängenbereich bezeichnet, dessen Grenzwerte auf das jeweils adressierte Lebewesen konkret anzupassen sind.

Außerhalb des sichtbaren Wellenlängenbereichs ist der ultraviolette Wellenlängenbereich angesiedelt. Ultraviolettes Licht in vernünftigen Dosen hat medizinisch positive Wirkung. Es ist aber auch aus der Fachliteratur genauso wie aus der Patentliteratur bekannt, dass der Sonnenbestrahlung der Effekt der aktinischen Keratose zugeordnet wird.

In dem Fachaufsatz "The Influence of Simulated Sunlight on the Inactivation of Influenza Virus in Aerosols", Michael Schuit et al. in "The Journal of Infectious Diseases", 378, JID 2020:221, wird die Auffassung vertreten, dass mit Sonnenlicht sowie mit simuliertem Sonnenlicht eine effektive Maßnahme gegen den Befall durch Influenza-Viren gefunden sei, wobei in dem Artikel ausgeführt wird, dass ein entsprechender UV-A- und UV-B-Anteil einen entsprechenden Einfluss entfalte.

Auch lassen sich in der Fachliteratur Stimmen finden, die erläutern, dass gewisse Wellenlängen im blauen Spektrum und/oder violetten Spektrum verschiedene positive Effekte beim Menschen hervorrufen können sollen.

So erläutert z. B. der Fachaufsatz "New Proof-of-Concept in Viral Inactivation: Virucidal Efficacy of 405 nm Light Against Feline Calicivirus as a Model for Norovirus Decontamination" von Rachael M. Tomb, Michelle Maclean, John E. Coia, Elizabeth Graham, Michael McDonald, Chintamani D. Atreya, Scott J. MacGregor und John G. Anderson, veröffentlicht am 31. Dezember 2016 in "Food and Environmental Virology", dass mit Hilfe eines Lichts der Wellenlänge 405 nm bakterizide, fungizide und auch viruzide Inaktivierungen durch oxidative Schädigung der Bakterien, Pilze und Viren herbeigeführt werden kann.

Der Fachaufsatz von der University of Arizona mit dem Titel "Blue light can help heal mild traumatic brain injury", veröffentlicht am 15. Januar 2020 auf Science Daily, schlägt vor, morgens eine Blaulichttherapie Leuten zuteilwerden zu lassen, die ein Gehirntrauma erlitten haben.

Aus verschiedenen Quellen lässt sich folglich ableiten, dass Blaulicht dem Gesundheitszustand von Menschen zuträglich ist, nicht zuletzt, wenn humoristische oder karnevalistische Aspekte hinzukommen.

Zur Erzeugung eines Blaulichtspektrums gibt es verschiedene Möglichkeiten. Ein Weg der Erzeugung eines blauen Farbspektrums ist mittels Halogenlampen mit Farbfiltern gegeben, die in einem geeigneten Temperaturbereich betrieben werden, wobei mittels des Farbfilters nur noch als einziges Farbspektrum Wellenlängen, die irgendwo im Frequenzband von 405 nm bis 500 nm liegen, durchgelassen werden. Ein anderer Weg der Erzeugung eines blauen Farbspektrums ist durch die Verwendung von Indiumgalliumnitrid oder Galliumnitrid als Halbleitermaterial für LEDs gegeben, wodurch diese zu Blaulicht-LEDs werden. Je nach Materialwahl und in Abhängigkeit von der Herstellungsgüte geben Hersteller von Leuchtmitteln, die für die Abgabe von blauem Licht bestimmt sind, unterschiedliche minimale und auch unterschiedliche durchschnittliche Betriebslebensdauern an, z. B. zum Teil nur 5.000 Betriebsstunden, aber auch gelegentlich bis zu 50.000 Betriebsstunden oder sogar mehr.

Geräte, die der Bestrahlung von Menschen mit Licht im sichtbaren Wellenspektrum, insbesondere mit einer ganz bestimmten Wellenlänge oder mit einem ganz bestimmten Frequenzband, dienen, werden schon seit vielen Jahrzehnten immer wieder in der Patentliteratur vorgestellt.

### Stand der Technik

Auf der einen Seite gibt es Lichtbestrahlungsgeräte, die entfernt an UV-Bestrahlungsgeräte aus dem Kosmetik- und Wellnessbereich erinnern; letztere Geräte, von denen die Lichtbestrahlungsgeräte ihrer Bauform nach abgeleitet sind, werden auch gemeinhin als typische Sonnenstudiogeräte bezeichnet. Ein solches Gerät lässt sich z. B. anhand der DE 20 2018 101 617 U1 (Inhaberin: RP-Technik GmbH; Bekanntmachungstag: 22.08.2019) studieren.

Auf der anderen Seite gibt es die eher durch den Begriff "kammerartig" zu bezeichnenden Geräte, die z. B. in der US 2019/054 310 A1 (Anmelder: William Woodburn; Veröffentlichungstag: 21.02.2019), in der US 2006/217 789 A1 (Erfinder: Thomas Perez; Veröffentlichungstag: 28.09.2006), in der EP 2 825 259 B1 (Inhaberin: National Biological Corporation; Erteilungstag: 07.06.2017) und in der EP 1878 467 B1 (Patentinhaberin: KBL Solarien AG; Erteilungstag: 01.10.2008) vorgestellt werden.

Weitere Gerätetypen der Kategorie Lichttherapiegerät lassen sich der US 10 478 635 B1 (Patentinhaberin: Joovv Inc.; Patenterteilungstag: 19.11.2019), der DE 200 13 335 U1 (Inhaberin: Biophoenix S.r.l.; Eintragungstag: 15.02.2001) sowie der WO 2015/150 126 A1 (Anmelderin: Koninklijke Philips N.V.; Veröffentlichungstag: 08.10.2015) entnehmen.

Die WO 2015/150 126 A1 stellt verschiedene Ausführungsformen vor; in einer Ausgestaltung gibt es einen Kontroller zur Steuerung der Leuchtmittel sowie ein über eine kabellose Verbindung angeschlossenes mobiles Gerät, um eine Benutzerschnittstelle anzubieten, durch die verschiedene Betriebsweisen der Lichttherapiekammer eingestellt werden können.

Selbst bei solchen Geräten wie dem nur einen Halbraum abdeckenden Panelen-Gerät, das in der EP 2 016 977 B1 (Patentinhaberin: Bartenbach Holding GmbH; Erteilungstag: 20.06.2012) beschrieben ist, gehen die jeweiligen Entwickler bzw. Hersteller davon aus, ein voluminöses Gerät schaffen zu müssen, damit ein Bestrahlungsgebiet oder Bestrahlungsraum ausgeleuchtet werden kann.

Interessanterweise konzentrieren sich viele Textersteller von Anmeldungstexten zum Thema "Lichttherapiekammer" auf die räumlichen Aspekte, wie die lichtabgebenden Teile zu gestalten sind. Im Vergleich hierzu weiterführende Aspekte und Themen, die von vielen Lichtdesignern eher als Randthemen angesiedelt werden, wie z. B. Regelung und Steuerung, Signalverarbeitung und wie besondere Aspekte zur Handhabbarkeit zu gestalten sind, werden in vielen Druckschriften äußerst peripher, wenn überhaupt, abgehandelt.

Allen zuvor aufgeführten Literaturstellen und den darin beschriebenen Gerätetypen von Lichttherapiegeräten ist gemein, dass diese Geräte, die aufgrund ihrer Aufgabe, ein Körperteil oder sogar einen Menschen zu umschließen, voluminös sind, als einstückige, in sich nicht trennbare Geräte zu realisieren seien.

Die US 4 469 102 A (Erfinder: Errol R. Fish; Patenterteilungstag: 04.09.1984) behandelt eine Solariumskammer, die möglichst keine Hautrötungen erzeugen soll und die deswegen nur Strahlen in einem Wellenlängenbereich von 320 nm bis 400 nm abstrahlen soll. Die sechseckige Kammer ist mit zwei Türflügeln ausgestattet. D. h., die einzelnen Paneele, von denen zwei Türen sind, stehen zueinander in einem 60° Winkel. In den Wänden der Paneele sind UV-Fluoreszenz-Lampen eingebaut, die hinter Plexiglasschirmen angeordnet sind, damit die Hitze der Lampen durch an dem Lampen hinter den Plexiglasschirmen vorbeiströmende Luftkühlung in einem Temperaturbereich unterhalb von 41 °C (105 °F) gehalten werden können. Wie eine solche UV-Solariumskammer aufstellbar sein soll, ist der US 4 469 102 A aber nicht zu entnehmen; das Dokument beschreibt ausschließlich die Situation der fertig aufgestellten Solariumskammer.

Ideen zur Montage einer wiederum sechseckigen UV-Solariumskammer, die ebenfalls im Wellenlängenbereich von 320 nm bis 400 nm Ultraviolette Strahlen aussenden kann, lässt sich der DE 27 25 179 A1 (Anmelderin: GTE Sylvania Inc.; Offenlegungstag: 22.12.1977) dahingehend entnehmen, dass die gesamte Kammer, die für eine Fotochemotherapie genutzt werden kann, nach einem Baukastenprinzip durch eine Anzahl von seitlichen Füllwänden aufzubauen ist, sodass handliche Einzelbauteile zur Verfügung stehen, was vorrangig auf Verwendung genormter Seitenwand-Bauteile zurückgeführt wird. Aus der Figurenbeschreibung ist aber auch wiederum zu entnehmen, dass die in den sechs Seitenwänden eingebauten 60 Leuchtstoffröhren eine so hohe Abwärme erzeugen, dass sowohl flammwidriger Kunststoff ABS als auch zusätzlich aufgeklebte UV-hemmende Deckschichten für die Seitenteile zu verwenden sind.

In der US 2005/240 248 A1 (Erfinder: Ralph Venuto SR; Patentveröffentlichungstag: 27.10.2005) wird eine weitere Solariumskammer mit Solariumslampen, genauer sechs übereinander angeordnete Lampen pro Seitenteil, in den sechs Seiten der Solariumskammer dargestellt.

Eine etwas andere Kammerform, nämlich eine der Zahl "8" nachempfundene Kammerform, ist der US 2005/065 578 A1 (Erfinder: Ralph Venuto SR; Patentveröffentlichungstag: 24.03.2005) zu entnehmen, wodurch ein Vorraum vor der eigentlichen Bestrahlungskammer geschaffen ist. In den Vorraum gelangt ein Nutzer durch Öffnen einer einflügigen Tür. Tritt der Nutzer in die eigentliche Bestrahlungskammer, kann er einen weiteren Flügel hinter sich schließen, damit er sich zwischen sechs Wandteile, die insgesamt eine geschlossene Hülle ergeben, stellen kann.

Die WO 2018/152 278 A2 (Anmelder: Scott Nelson et al.; Veröffentlichungstag: 23.08.2018) bebildert ihre Beschreibung von Beleuchtungseinheiten auf LED-Basis mit über Flaschenzüge parallel zu Wänden aufgehangen Kästen, die die Beleuchtungseinheiten darstellen.

Aus vielen der oben zitierten Druckschriften kann ein kundiger Leser einen gewissen Aufwand bei der Montage und bei der Aufstellung solcher Solarienkammern bzw. Lichttherapiekammern "herauslesen". Bei Kammern, die mit Fluoreszenz-Lampen arbeiten, sind deren Lampen in der Regel gesondert zu transportieren. Die Lampen können erst nach fertiger Aufstellung der gesamten Kammer in ihre Lampenfassungen eingesetzt werden, weil schon leichte Erschütterungen und Schläge (z. B. beim Umwuchten einzelner Module) bei diesem Typ Leuchtmittel häufig zu irreparablen Schäden führen.

Zwei Solariumskammernbeschreibungen, die die dislozierte bzw. die in der Ferne angesiedelte "User-Profil"-Archivierungen, z. B. in der "cloud", zur Steuerung der Lichtprofile und der Beleuchtungsintensität vorschlagen, stellen die US 2013/172 963 A1 (Anmelderin: Benesol Inc.; Patenterteilungstag: 04.07.2013) und die WO 2019/118 777 A1 (Anmelderin: Benesol Inc.; Veröffentlichungstag: 20.06.2019) dar. In dem Wissen über die schädigende Wirkung von UV-Strahlen soll zentral berechnet werden, welche Lichtdosen einem sich eindeutig verifizierenden Nutzer einer entsprechenden Solariumskammer noch zugeführt werden darf.

Ein mobiles, zur Heilungsunterstützung dienendes, drei Leuchtflächen umfassendes Gerät, das um eine Körperextremität gebunden werden kann, wird in der US 2019/240 501 A1 (Anmelderin: 9369201 Canada Inc.; Veröffentlichungstag: 08.08.2019) beschrieben. Ein solches Gerät soll über eine Netzwerkanbindung mit einem Therapiesteuerserver verbunden sein, damit ein auf die beabsichtigte Heilung angepasstes Beleuchtungsprofil dem mobilen Gerät aufgespielt werden kann.

Die zuvor genannten Druckschriften gelten mit ihrer Benennung als vollumfänglich in vorliegende Erfindungsbeschreibung inkorporiert. Hierdurch soll vermieden werden, nicht mehr erneut und wiederholt allgemein bekannte Zusammenhänge zwischen Farbtönen, Wellenlängen, räumlichen Gestaltungen, Lichtabgabeflächen und dazugehörigen Lichtquellen erörtern zu müssen, sondern durch Verweis auf die Druckschriften als ebenfalls definiert für vorliegende Erfindung ansehen zu dürfen.

### Aufgabenstellung

Obwohl ausweislich der oben zitierten Schutzrechtsliteratur wenigstens seit ca. 15 bis 20 Jahren an den unterschiedlichsten Formen von Lichttherapiekammern gearbeitet wird, ist bis heute keine wirkliche, weitläufige und allgemeine Verbreitung solcher Kammern in Europa feststellbar. Dies mag auch daran liegen, dass die bisher bekannten Vorschläge für Lichttherapiekammern von der Idee der stationären Anlagen, vorzugsweise als stationäre Krankenhausgeräte, geleitet zu sein scheinen. Bei einer Ausstattung von Krankenhäusern mögen Kriterien wie Zuverlässigkeit, Dauerbetriebsfähigkeit und mechanische Stabilität im Vordergrund stehen, was aber für eine Breitenversorgung nicht in dem Maße notwendig ist.

Für eine Breitenversorgung mit Lichttherapiekammern dürften im Vergleich mit Krankenhaus-Geräten andere technische Kriterien eher den Ausschlag geben. Lichttherapiekammern, die einen hohen Gebrauchswert bei einfacher Handhabbarkeit aufweisen, dürften vermutlich eher Anklang bei Privatnutzern finden können als die für eingewiesenes medizinisches (Fach-)Personal bestimmten Krankenhausgeräte.

Generell wäre es daher vorteilhaft, ein Prinzip, einen konstruktiven Ansatz bzw. ein Design für eine Gestaltung bzw. für Ausführungsmöglichkeiten von Lichttherapiekammern zu kennen, die eher außerhalb von Krankenhäusern Zustimmung finden. Hierbei kann der Blick auf solche Kriterien wie die leichte Bedienbarkeit gerichtet werden. Auch kann der Blick auf das Potential gerichtet werden, kurzfristig eine angepasste, reparierte und gegebenenfalls auch wieder demontierte Lichttherapiekammer zu schaffen, um an einer anderen Stelle genauso leicht wieder die Lichttherapiekammer aufbauen zu können.

### Erfindungsbeschreibung

Die erfindungsgemäße Aufgabe wird durch eine Lichttherapiekammer nach Anspruch 1 gelöst, ein geeignetes Verfahren zur Überwachung einer Lichttherapiekammer lässt sich Anspruch 14 entnehmen. Vorteilhafte Weiterbildungen lassen sich den abhängigen Ansprüchen entnehmen.

Eine Lichttherapiekammer ist dafür bestimmt, ein Lebewesen, wie z. B. einen Menschen, in ihrem Inneren aufzunehmen, um auf das sich im Inneren der Lichttherapiekammer aufhaltende Lebewesen eine kontrollierte Dosis von elektromagnetischen Wellen, z. B. eine Zusammensetzung ausgewählter Lichtwellenlängen im sichtbaren Wellenlängenbereich, einwirken zu lassen. Die Lichttherapiekammer ist also eine Zelle bzw. ein schließbarer Raum, der also verschlossen oder auch geöffnet werden kann. Der Raum ist so dimensioniert, dass er einen Menschen aufnehmen kann. Die Lichttherapiekammer umschließt ein Volumen, z. B. zwei Kubikmeter, in dem sich ein Mensch, vorzugsweise in einer komfortablen Körperhaltung, aufhalten kann. Zur angenehmeren Verweildauer im Inneren der Lichttherapiekammer kann diese mit einer Sitzgelegenheit (Stuhl) oder einer Liegegelegenheit (Liege) ausgestattet sein, auf der sich der zu bestrahlende Mensch niederlassen kann.

Die Lichttherapiekammer kann in einer Ausgestaltung so ausgeführt sein, dass sie das Licht des Innenraums nicht nach außen austreten lässt, sobald die Tür der Lichttherapiekammer geschlossen ist. In einer hierzu alternativen Ausgestaltung kann die Lichttherapiekammer einen Teil, insbesondere einen äußerst geringen Teil wie z. B. weniger als 1 % des Lichtstroms aller Leuchtmittel, nach außen abgeben, um z. B. den Betrieb zu signalisieren oder um z. B. den gerade im Inneren der Lichttherapiekammer vorherrschenden Farbton anzuzeigen.

Es ist besonders günstig, wenn die Lichttherapiekammer von einer elektronischen Einheit gesteuert wird, die auf dem Dach bzw. auf der obersten Decke der Lichttherapiekammer angeordnet sein kann. Diese elektronische Steuerungs- und Überwachungseinheit ist dafür da, Lichtquellen der Lichttherapiekammer ein-, aus- und umzuschalten und idealerweise ihren Betrieb zu überwachen. Diese Steuerungs- und Überwachungseinheit kann als erste Einheit bezeichnet werden, insbesondere weil sie unmittelbar in der Nähe des Innenraums der Lichttherapiekammer, z. B. außen an dem die Lichttherapiekammer ausmachenden Gehäuse, angesiedelt ist.

Besonders vorteilhaft sind Leuchtmittel in den Lichtquellen, die LEDs sind, weil diese - neben vielen weiteren Vorteilen - eine hohe Lichtausbeute pro einzusetzendem elektrischen Strom bieten (Maßeinheit: Lumen/Watt). Leuchtmittel mit ähnlich günstigen Wirkungsgraden sind OLEDs. Auch einige Ausführungsformen von Natriumdampflampen können hohe Wirkungsgrade erreichen. Die einzelnen Leuchtmittel können auf einem Träger zu Gruppen zusammengeschlossen sein. So können LEDs auf LED-Streifen angesiedelt werden. Ein LED-Streifen trägt mehrere LEDs.

Zwischen der Steuerungs- und Überwachungseinheit und den Lichtquellen bzw. ihren Leuchtmitteln, z. B. in Gestalt von LED-Streifen, sollte es eine elektronische Verbindung geben, über die die Leuchtmittel, wie z. B. die LEDs, ein- und ausgeschaltet werden können. Vorteilhafterweise können zusätzliche Funktionsüberwachungen von der Steuerungs- und Überwachungseinheit durchgeführt werden.

Die Lichttherapiekammer ist vorteilhafterweise mit einem Modul ausgestattet, über das die Lichttherapiekammer eine Internetverbindung herstellen kann, z. B. über eine Mobilfunkverbindung wie z. B. über eine LTE-Verbindung. Andere Möglichkeiten der Internetverbindung bieten in der Steuerungs- und Überwachungseinheit integrierte Router oder Modems, sodass über ein solches Modem eine Internetverbindung, insbesondere über für die Öffentlichkeit zur Verfügung stehende Leitungen wie z. B. VDSL-Leitungen, hergestellt werden kann.

Dem gesamten System zuträglich ist es, wenn sich an einer anderen Stelle im Internet eine zweite Steuereinheit befindet, mit der die erste Steuerungs- und Überwachungseinheit eine Kommunikation durchführen kann. Über die Internetverbindung wird eine Verbindung zwischen erster Steuerungs- und Überwachungseinheit und zweiter Steuereinheit hergestellt.

Die zweite Steuereinheit kann so gestaltet sein, dass sie sich u. a. der Aufgabe widmen kann, Archivierungen von Daten zu der Lichttherapiekammer durchzuführen. Hierzu werden Daten von der ersten Steuerungs- und Überwachungseinheit an die zweite Steuereinheit übertragen, damit die zweite Steuereinheit die empfangenen Daten speichern kann.

In Reaktion auf einzelne Daten, auch Datenmuster können als Kriterium für eine Reaktion berücksichtigt werden, der Lichttherapiekammer kann vorgesehen sein, dass die zweite Steuereinheit einen Eingriff auf die erste Steuerungs- und Überwachungseinheit durchführen, z. B. aufgrund von Messdaten der ersten Steuerungs- und Überwachungseinheit. Hierdurch kann der Betrieb der Lichttherapiekammer beeinflusst werden (z. B. können so geartete Vorgänge unternommen werden wie: ein Dimmen, ein Ausschalten, eine Durchführung einer Helligkeits(-nach-)regelung, eine Anpassung der, insbesondere maximalen, Betriebsdauer usw.). Vorteilhaft kann es sein, wenn mit zunehmender Betriebsdauer der Strom an bzw. für die Leuchtmittel nach und nach erhöht wird. Alterungserscheinungen können so ausgeglichen werden. Obwohl die Leuchtkraft der Leuchtmittel abnimmt, bleibt die Helligkeit in der Lichttherapiekammer gleich.

Ist die zweite Steuereinheit so gestaltet, dass sie mehrere Lichttherapiekammern verwalten und betreuen kann, so können Informationen einer ersten Lichttherapiekammer mit Informationen einer zweiten Lichttherapiekammer abgeglichen werden, z. B. können Betriebszeiten einer ersten Lichttherapiekammer mit Betriebszeiten einer zweiten Lichttherapiekammer und daraus resultierende Wartungszeitpunkte und Reparaturmaßnahmen abgeleitet werden.

Vorteilhaft ist ein Verfahren, mit dem die Lichttherapiekammer überwacht werden kann. Hierbei kann eine Überwachungseinheit eine Funktionstüchtigkeit von Leuchtmitteln der Lichttherapiekammer durchführen. Bei einem Ausfall, bei einer Verschlechterung und/oder einem unerwünschten Betriebszustand der Leuchtmittel kann eine Wartung, eine Reparatur und/oder eine Besichtigungsbeauftragung an eine Serviceperson oder an einen Servicebetrieb ausgelöst werden. Stellt die erste Steuer- und Überwachungseinheit oder die zweite Steuereinheit, insbesondere aufgrund ihrer archivierten Daten, fest, dass der Betrieb der Lichttherapiekammer zu unterbinden ist oder von den gewünschten Betriebsweisen abweicht, so kann wenigstens eine der beiden Einheiten den Betrieb der Lichttherapiekammer beeinflussen.

Nachfolgend werden vorteilhafte Ausgestaltungen und Weiterbildungen dargelegt, die für sich gesehen, sowohl einzeln als auch in Kombination, ebenfalls erfinderische Aspekte offenbaren können.

Die beiden Geräte "zweite Steuereinheit" und "erste Steuerungs- und Überwachungseinheit" können zueinander wie ein Mastergerät und ein Slavegerät realisiert sein. Das Slavegerät kann z. B. durch das Mastergerät steuerbar sein. Bietet das Slavegerät eine besondere Schnittstelle für die Kommunikation mit dem Mastergerät, so kann über die Schnittstelle eine Einflussnahme für das Mastergerät eingeräumt werden. Beispielsweise kann das Mastergerät z. B. derart gestaltet sein, dass es das Slavegerät an einem Betrieb hindert. Besonders vorteilhaft ist es, wenn die zweite Steuereinheit als Mastergerät ausgestaltet ist. In diesem Fall ist die erste Steuerungs- und Überwachungseinheit ein Slavegerät.

Die Lichttherapiekammer kann ein einziges oder auch mehrere Zeitmodule umfassen. Auch kann eine Messvorrichtung vorgesehen sein.

Vorteilhaft ist es, wenn Daten zu Betriebszeiten, z. B. von gleichartigen Leuchtmitteln, oder Daten von der Art und Weise des Betriebs der Leuchtmittel gespeichert werden. So können z. B. die einzelnen Farbphasen von RGB-LEDs in einem Speicher abgelegt werden. In einem Speicher ist gespeichert, zu welchem Zeitpunkt welche LEDs welchen Farbton abgestrahlt haben. Solche Daten lassen sich nach dem Messen, Ermitteln und Speichern über eine Verbindung zur zweiten Steuereinheit übertragen. Hat die zweite Steuereinheit Zugriff auf einen größeren Speicher, z. B. weil er als Cloud-Speicher (nahezu) unbegrenzt ist, so können zu längeren Zeiträumen Daten und ggf. auch mehr Daten zu der Lichttherapiekammer abgelegt werden.

Besonders vorteilhaft ist es, wenn die zweite Steuereinheit nicht unmittelbar neben der Lichttherapiekammer angeordnet ist, sondern von dieser Lichttherapiekammer entfernt platziert ist. Die zweite Steuereinheit kann die Aufgabe eines Zentralrechners übernehmen, der sich, wie bereits angesprochen, z. B. in der Cloud befinden kann. Über ein solches, idealerweise von mehreren Stellen aus bedienbares Gerät, z. B. als virtuelles Gerät, kann auf die erste Steuerungs- und Überwachungseinheit eingewirkt werden. Somit ist der Zentralrechner ein Gerät für eine Fernsteuerung der Lichttherapiekammer. Die zweite Steuereinheit befindet sich von der Lichttherapiekammer entfernt, also disloziert an einem, z. B. replizierbaren, Ort.

Die erste Steuerungs- und Überwachungseinheit ist als Gerät zur Prüfung der Funktionstüchtigkeit einzelner Komponenten der Lichttherapiekammer gestaltbar. Z. B. kann die erste Steuerungs- und Überwachungseinheit mit einem Überwachungsschaltkreis ausgestattet sein, der die Funktionstüchtigkeit einzelner (im Sinne einer Lichtquelle für alle; im Sinne einer Stichprobe) oder aller Lichtquellen kontrollieren kann. So ist es möglich, die Lichttherapiekammer auf Funktionstüchtigkeit mit Hilfe des Überwachungsschaltkreises zu überwachen. Hierbei kann die Überwachung nur anhand einiger der Lichtquellen erfolgen. In einer alternativen Ausführungsform können auch alle Lichtquellen überwacht werden.

Eine weitere Möglichkeit, wie die Funktionstüchtigkeit der Lichtquellen anhand ihrer Leuchtmittel überwacht werden kann, ist durch eine Betriebsstrommessung gegeben. In einer Messschaltung werden die Leuchtmittel selbst messtechnisch überwacht. Die Leuchtmittel, die in den Lichtquellen das Licht produzieren, können besonders gut auf Funktionstüchtigkeit überwacht werden.

Sind mehrere Leuchtmittel, z. B. RGB-LEDs, in Serie verschaltet, so lässt sich der Ausfall eines Leuchtmittels der in Serie verschalteten Leuchtmittel anhand eines nicht fließenden Betriebsstroms (trotz anliegender Spannung) messen. Auch können Fehlersignalgeneratoren solche Messungen ermöglichen. So ist es möglich, bei einer Unterschreitung oder Überschreitung eines erwarteten Betriebsstroms ein Signal an die erste Steuerungs- und Überwachungseinheit zu senden. Liegt außerhalb eines akzeptablen Betriebsstrombereichs der gemessene Betriebsstrom, kann ein entsprechendes Signal an die erste Steuerungseinheit (erste Steuerungs- und Überwachungseinheit) und/oder an die zweite Steuereinheit gesendet werden. Als Fehlersignalgeneratoren eignen sich Hochfrequenzsignalgeneratoren, die ein überlagertes Messsignal auf eine Leitung aufprägen können, um anhand seiner Dämpfung und/oder seiner Reflexion den Zustand einer oder mehrerer Lichtquellen zu messen.

Eine weitere Möglichkeit der Überwachung (und sogar der Prognose von Restbetriebszeiten) ist durch die Summation von Einzelbetriebszeiten gegeben. Kann ein Überwachungsschaltkreis eine kumulierte Betriebszeit errechnen oder ermitteln, kann - auf Basis von üblichen maximalen Betriebszeiten - die zu erwartende Restlaufzeit (Restlebensdauer) für einzelne Lichtquellen bzw. ihre Leuchtmittel, z. B. für LEDs, abgeschätzt werden. Nähert sich der Betrieb einem solchen Endwert, so kann eine Warnmeldung von der ersten Steuerungs- und Überwachungseinheit an die zweite Steuereinheit übermittelt werden.

Daten, die von der ersten Steuerungs- und Überwachungseinheit an die zweite Steuereinheit übermittelt werden können, können z. B. Gesamtbetriebszeiten oder auch Einzelbetriebszeiten sein. Summierte Betriebszeiten können nach den einzelnen Farbtönen, die von den Lichtquellen ausgestrahlt worden sind, aufgeschlüsselt werden bzw. aufgeteilt werden. Sind mehrere Farbtöne auswählbar, so kann eine bevorzugte Nutzung eines der zur Verfügung stehenden Farbspektren dazu führen, dass die für die Aussendung dieses Farbspektrums zuständigen Lichtquellen, z. B. Rotlicht-LEDs, sich früher einem Betriebszeitende annähern als die anderen LEDs. Stehen mehrere Farbtöne zur Verfügung, die im sichtbaren Wellenlängenbereich liegen, so kann nach den einzelnen Wellenlängen aufgeschlüsselt eine Einzelbetriebszeit und eine Gesamtbetriebszeit in der zweiten Steuereinheit gesammelt und gespeichert werden. Beispielsweise kann so eine Aufschlüsselung eine Betriebszeit für Rot, eine Betriebszeit für Grün und eine Betriebszeit für Blau zu jedem Leuchtmittel auflisten. Diese Daten können bei jeder Betriebsaufnahme der Lichttherapiekammer aktualisiert werden. D. h., die Daten werden permanent mitgeschrieben und aktualisiert. Vereinfachtere Varianten dokumentieren nur eine Gesamtbetriebszeit aller Leuchtmittel und sehen davon ab, die Betriebszeit für jedes Leuchtmittel einzeln zu dokumentieren.

Überwachungen gestalten sich besonders einfach, wenn mehrere Leuchtmittel bzw. mehrere Lichtquellen in einer Reihenschaltung betrieben werden. Üblich ist es z. B. LED-Streifen mit mehreren in Reihe geschalteten LEDs zu realisieren. Für eine Aufrechterhaltung der Funktionstüchtigkeit mag es aber auch vorteilhaft sein, wenn eine Parallelschaltung zur Anordnung von mehreren Lichtquellen in Reihe gegeben ist. So ist es z. B. möglich, eine erste Gruppe von in Reihe geschalteten Lichtquellen parallel zu einer zweiten Gruppe von in Reihe geschalteten Lichtquellen anzuordnen. Die Lichtquellen können in jeder Gruppe gleich viele sein, z. B. fünf oder zehn LEDs, die die verwendeten Leuchtmittel darstellen. Fällt die eine Gruppe aus, so kann immer noch die andere Gruppe weiterbetrieben werden. Hierdurch wird aber die Lichtintensität (üblicherweise) verringert (sofern nicht die elektrischen Ströme in solchen Phasen erhöht werden). Misst die Überwachungseinheit bzw. der Überwachungsschaltkreis die Helligkeit innerhalb der Lichttherapiekammer oder die abgestrahlte Helligkeit von einer Bestrahlungsgroßfläche, so können solche punktuellen Ausfälle von mehreren in Reihe verschalteten Lichtquellen (Leuchtmittel) identifiziert werden.

Eine weitere Möglichkeit der Überprüfung und Einflussnahme besteht darin, eine Stromsteuerung des elektrischen Stroms für die Lichtquellen durchzuführen. Werden alle Lichtquellen (genauer gesagt die Leuchtmittel) zeitgleich gedimmt, so sollte die Überwachungsschaltung diesen Vorgang detektieren können. Auch gibt es die Möglichkeit, einen Funktionsablauf (z. B. eine Signalisierung durch Helligkeitsschwankungen), durch den die Betriebsdauer signalisiert wird, zugleich bzw. ergänzend für einen Überwachungsalgorithmus oder Überwachungsvorgang zu nutzen.

Eine weitere Möglichkeit der Überwachung der Bestrahlungsgroßflächen ist durch eine Spannungsmesseinheit gegeben, die eine Spannung, z. B. einen Spannungsabfall an einem Vorwiderstand für z. B. LEDs, misst. Befinden sich mehrere solcher Leuchtmittel auf einem gemeinsamen Träger, kann anhand des Spannungsteilungsverhältnisses oder anhand des Spannungsabfalls die Funktionstüchtigkeit der von der ersten Steuerungs- und Überwachungseinheit überwachten Lichtquelle festgestellt werden. Eine Überwachung mehrerer Lichtquellen (oder mehrerer Leuchtmittel) ist gegeben.

Eine weitere Möglichkeit der Überwachung der Funktionstüchtigkeit der Lichttherapiekammer bietet sich durch die Messung einer Homogenität einer Lichtfläche im Inneren der Lichttherapiekammer. Werden mehrere LED-Streifen mit gleichen (elektrischen) Strömen betrieben, so sollten, sofern die LED-Streifen mit den gleichen Toleranzen gefertigt sind, die LED-Streifen gleich hell sein.

Eine der Steuerungs- und Überwachungseinheiten, insbesondere die erste Steuerungs- und Überwachungseinheit, kann eine Einheit für eine Helligkeitsmessung umfassen. Vorteilhaft ist es, wenn auch eine Auswerteeinheit vorhanden ist. Eine solche Auswerteeinheit kann z. B. auswerten, ob eine Abweichung eines Helligkeitswerts von einem vorbestimmten Helligkeitswert eingetreten ist. Wird anhand des gemessenen Helligkeitswerts festgestellt, dass eine außergewöhnliche Situation vorliegen könnte, können weitere Funktionstüchtigkeitsuntersuchungen durchgeführt werden, z. B. Spannungsmessungen, Stromsteuerungen, Strommessungen, Homogenitätsmessungen, Reihenschaltungsmessungen, Fehlersignalmessungen und Betriebsstrommessungen. In diesem Zusammenhang ist es verständlich, dass nicht alle zuvor genannten Messungen, Maßnahmen und Untersuchungen von einer Anlage, einem Überwachungsschaltkreis oder eine Lichttherapiekammer durchzuführen sind, sondern in Abhängigkeit von den Gegebenheiten die eine Messung vorteilhafter sein kann als die andere Messung. Soll die Lichttherapiekammer besonders einfach konzipiert sein, so gibt es nur eine einzige Messung, die eine Störung der Funktionstüchtigkeit ermittelt. Soll ein Servicetechniker vor seinem Besuch möglichst umfassend über den Zustand einer Lichttherapiekammer aufgeklärt werden, so mag es vorteilhaft sein, möglichst viele Messungen autark in der Lichttherapiekammer durchführen zu lassen.

Weitere vorteilhafte Überwachungsfunktionen können mithilfe der Steuerungs- und Überwachungseinheit in einer Lichttherapiekammer implementiert sein. Jede der Überwachungsfunktionen kann eigenständig oder in Kombination mit weiteren Überwachugsfunktionen Teil der Lichttherapiekammer sein.

Zur Überwachung der Lichttherapiekammer eignen sich Helligkeitsmessungen oder Helligkeitsprüfungen. Mithilfe einer Helligkeitsmessung oder einer Helligkeitsprüfung kann der Betrieb der Leuchtmittel nachgeregelt werden.

Zur Überwachung kann eine Strommessung, insbesondere eines Gesamtstroms, durchgeführt werden. Wird daran gedacht, dass ein wesentlicher Faktor der Betriebskosten durch die Kosten für den elektrischen Strom verursacht werden, kann mit einer Stromsteuerung nicht nur die Alterung der Leuchtmittel verlangsamt werden, sondern auch die Betriebskosten können reduziert werden.

Besonders interessant ist ein Betriebsstundenzähler.

Sind Ventilatoren oder Lüfter in der Lichttherapiekammer verbaut, so können die Betriebsweisen und/oder die Funktionstüchtigkeit überwacht werden.

Hat die Lichttherapiekammer solche Sensoriken wie eine Personenpräsenzüberwachung, eine Personenaktivitätsüberwachung, eine Personenherzschlagsüberwachung und/oder eine Personenlebendüberwachung, so kann der Betrieb der Leuchtmittel an die im Inneren der Lichttherapiekammer vorhandenen Person angepasst oder abgestimmt werden.

Wie zuvor schon angesprochen, altern Leuchtmittel. LEDs lassen im Laufe der Zeit bei ihren Helligkeiten nach. Idealerweise bemerkt kein Nutzer einer Lichttherapiekammer, ob die Lichttherapiekammer mit betagten oder mit neuen Leuchtmitteln ausgestattet ist.

Eine weitere Überwachungsfunktion kann die Überwachung der Stellung eines oder mehrerer Türflügel sein.

Eine Lichttherapiekammer, die eine entsprechende erste Steuerungs- und Überwachungseinheit umfasst, die vorteilhafterweise mit einer zweiten Steuereinheit kommunizieren kann, kann anhand von prognostizierenden Restbetriebszeiten für einzelne Leuchtmittel oder für alle Leuchtmittel im Vorhinein bestimmen, wann geeignete Wartungszeitpunkte bzw. Teile- und Komponentenreparaturzeitpunkte erreicht sein werden. Dank solcher vorausschauenden Schaltungen und Algorithmen ist es möglich, ohne längere Unterbrechungszeiten eine Funktionstüchtigkeit der Lichttherapiekammer zur Verfügung zu stellen.

Auf Basis der vorgestellten Lichttherapiekammer und dank des dazugehörigen Verfahrens ist es möglich, sogar ein selbstlernendes System, das vorzugsweise in der zweiten Steuereinheit vorgehalten wird, zu entwickeln. Die Betriebszeiten der Leuchtmittel sind üblicherweise Richtwerte (angegeben werden üblicherweise Werte wie 2.000 Stunden, 3.000 Stunden, 50.000 Stunden), die von Herstellern der Leuchtmittel angegeben werden. Die Betriebszeiten werden von den Herstellern berechnet und in ihren Laborumgebungen verifiziert. In der Praxis hat es sich aber gezeigt, dass die angegebenen Betriebszeiten der Leuchtmittel häufig sehr stark von den jeweiligen Herstellerangaben abweichen, weil z. B. die Umgebungstemperaturen, die Betriebstemperaturen, die Einschalthäufigkeiten, die Dimmhäufigkeit und die Varianz im Betriebsstrom nicht in den Berechnungen berücksichtigt werden, zumindest nicht in Übereinstimmung mit dem tatsächlichen üblichen Betrieb bei den Berechnungen mitberechnet worden sind. Zeigt es sich z. B. anhand von Betriebsmustern, dass eine bestimmte Betriebsweise zu längeren und eine andere Betriebsweise zu kürzeren Lebensdauern führen, so kann die zweite Steuereinheit auf die erste Steuerungs- und Überwachungseinheit einwirken, um z. B. besonders nachteiliges Verhalten und eine besonders nachteilige Betriebsweise (z. B. häufige kurzphasige Betriebe von jeweils weniger als 1 Minute) erst gar nicht zuzulassen. Im Laufe der Zeit, insbesondere wenn mehrere Lichttherapiekammern an verschiedenen Orten betrieben werden, kann der Richtwert eines Herstellers von Leuchtmitteln nach einiger Zeit auf die tatsächlich zu erwartende Betriebsdauer angepasst bzw. korrigiert werden. Zu einer Lichttherapiekammer sind Wartungszeitpunkte ausweisbar, die mit den angeblich möglichen Betriebslebensdauern der Leuchtmittel nur noch eine geringe Konkordanz haben.

Weitere interessante Aspekte werden nachfolgend vorgestellt:
Eine Kammer, in der ausgewählte Lichtverhältnisse, z. B. durch eine ganz bestimmte Lichtintensität, eine ganz bestimmte Beleuchtungsstärke, eine ganz bestimmte Helligkeit und/oder eine Zusammenstellung ausgewählter Lichtwellenlängen (z. B. eine Kombination von ausschließlich vorhandenen, d. h. ganz bestimmten Wellenlängen wie z. B. eines Lichts mit den beiden Wellenlängen 415 nm und 453 nm oder wie z. B. Licht nur einer einzigen Wellenlänge wie z. B. von nur 450 nm), herrschen, kann als Lichttherapiekammer bezeichnet werden, insbesondere unter dem Blickwinkel, dass sich Personen in dieser Kammer für eine gewisse Zeit, z. B. für 15 Minuten oder auch für 30 Minuten, aufhalten, um sich dem ausgewählten Lichtspektrum bzw. den besonderen Lichtverhältnissen auszusetzen.

Wie einleitend festgehalten, kann Licht ausgewählter Wellenlängen (z. B. im Bereich von 420 nm, z. B. im Bereich von 540 nm oder z. B. im Bereich von 580 nm) dazu verwendet werden, den Gemütszustand einer Person positiv zu beeinflussen. Wie aus den medizinischen Untersuchungen, die weiter oben vorgestellt worden sind, zu entnehmen ist, kann Licht ausgewählter Wellenlängen auch dazu genutzt werden, als Alternative zu einem Antimykotikum (anti-fungizid), anti-bakteriell und/oder anti-viral auf eine sich in der Lichttherapiekammer aufhaltende Person oder Lebewesen einzuwirken. Die Lichttherapiekammer ist dafür vorgesehen, eine bestimmte Dosis eines Lichts in ihrem Inneren während einer bestimmten Zeit zur Verfügung zu stellen. Die Dosis kann sowohl in Bezug auf die Lichtenergie als auch in Bezug auf die Dauer der Bestrahlung eingestellt werden. Vorteilhafterweise wird das Licht durch elektrische bzw. elektronische Strom-Licht-Wandler wie LEDs erzeugt. In einer vorteilhaften Weiterbildung der Lichttherapiekammer kann diese so gestaltet sein, dass überhaupt nur Licht in einem sichtbaren Wellenlängenbereich in der Lichttherapiekammer zur Verfügung steht.

Vorteilhaft ist es, wenn die Lichttherapiekammer als eine umschließende Hülle mit einem in ihrem Inneren angeordneten Zentralbereich gestaltet ist, der für ein Eintreten eines Lebewesens in ihn gestaltet ist. Das in der Lichttherapiekammer erzeugte Licht soll einen als Lichtbestrahlungsraum gedachten Bereich im Inneren der Lichttherapiekammer ausleuchten. Hierfür kann Licht von der Lichttherapiekammer so abgestrahlt werden, dass es in den Zentralbereich gelangt.

Ein Panel (vgl. das englische Wort "panel") einer Lichttherapiekammer unterscheidet sich von dem im Deutschen gebräuchlichen Wort "Paneele" dahingehend, dass Paneelen als ein Element einer Wand- oder Deckenverkleidung keine freistehenden Körper sind. Ein Panel ist ein freistehender Körper, der eine Umwandlungsvorrichtung umfasst, die zwischen elektromagnetischen Wellen und elektrischem Strom wandeln kann, z. B. eine Quelle für Licht aufweist, das von einer Fläche bzw. von einer Flachseite des Panels ausgeht. Mehrere lichtabstrahlende Elemente bzw. Bereiche bilden ein Strahlungsfeld. In Anlehnung an das englische Wort "panel" kann als Plural das Wort "Panels" oder (auch) eingedeutscht das Wort "Panele" - in bedeutungsidentischer Weise - verwendet werden.

In der deutschen Sprache ist der Begriff "Panele" auch dafür bekannt, dass diese Elemente, zumeist einzeln, zur Darstellung von Licht-Bildern, wie Displays, genutzt werden. Bei derartigen Panelen wird aber Wert auf die Farbtreue der Darstellung gelegt, wohingegen die Panele gem. der vorliegenden Erfindung ganz bevorzugt eine Lichtintensität in einem vorgegebenen Wellenlängenbereich bereitstellen. Wenn die Panele einer Lichttherapiekammer modulartig ausgebildet sind, können mehrere Panele miteinander zu einer gemeinsamen Lichtabstrahlungsfläche zusammengeschlossen werden, die auch als Großfläche bezeichnet werden kann. Modulartige Panele weisen vorzugsweise elektronische und insbesondere mechanische Verbindungselemente zum gegenseitigen Aneinanderschließen an Schmalseiten auf. Damit ist ein erstes Modul mit einem zweiten Modul (usw.) verbindbar.

Panele können außerdem so ausgebildet sein, dass sie in gegenüberliegende Halbraumwinkelbereiche, also in Lichtabstrahlungsachsen, die vom Ort der Lichtquelle ausgehend entgegengesetzt orientiert sind, Licht abgeben. Zur Begrenzung des Lichtraums wird aber von einem erfindungsgemäßen Panel nur ein Halbraumwinkelbereich bestrahlt, sodass von einer Lichtabstrahlungsachse im Sinne von einer Halbachse zu sprechen ist. Damit ist mithilfe von Panelen ein innerer Lichtraum und ein äußerer, nicht oder nur unwesentlich durch Streulicht bestrahlter Raum ausbildbar. Streulicht kann sich bekanntlich an Partikeln (wie Staub), Schichten (wie Fingerabdrücke) oder Oberflächen (wie Haut) bilden, die in einem Strahlengang einer Lichtquelle liegen bzw. gelangt sind.

Im Zentrum der Lichttherapiekammer verläuft nun eine Lichtbestrahlungsraumachse. Die Lichtabstrahlungsachsen, die im Wesentlichen senkrecht von den Panelen weggeführt sind, und die Lichtbestrahlungsraumachse schneiden sich, vorzugsweise in einem 90° Winkel.

Idealerweise ist die Lichttherapiekammer aus gleichartigen Panelen als Wandelementen zusammengesetzt (plattenartige bzw. scheibenartige Elemente bzw. Körper zur Lichtabgabe). Diese Panelen sind modulartig gestaltet. Die Panelen können auch als Module bezeichnet werden. Mehrere einzelne Panele werden jeweils als Modul verwendet, um aus mehreren Modulen eine Wand der Lichttherapiekammer zusammenzusetzen.

Neben den Panelen bzw. den Modulen sollte es auch einen Deckel geben, der die Lichttherapiekammer nach oben abdeckt. Werden die Module zusammengefügt und wird auf den Modulen von oben (im Sinne von oben darauf) der Deckel montiert, so ergibt sich eine vom Boden aufragende, geschlossene Zelle. In diesem Fall sind die einzelnen Panele nebeneinander angeordnet und schaffen so ein Vieleck. Eine besonders vorteilhafte Form für die Lichttherapiekammer ist ein Achteck. Achtecke und ähnliche Polyeder zeichnen sich durch eine gute Ausleuchtung aus, wenn Lichtquellen auf ein Zentrum des Polyeders gerichtet sind.

Im Inneren der Lichttherapiekammer, dort wo sich der Zentralbereich befindet, kann also eine (gedachte) Lichtbestrahlungsraumachse durch die Lichttherapiekammer "geschlagen" bzw. "gezogen" werden. Dort, wo die Lichtbestrahlungsraumachse verläuft, ist der Zentralbereich so gestaltet, dass eine Person in jenem Bereich wenigstens stehen kann, vorteilhafterweise sich auch hinsetzen kann, um während der Dauer einer Bestrahlung (z. B. während eines Zeitraums von ca. 20 Minuten) entspannt, z. B. in sitzender Weise sich der Bestrahlung auszusetzen. Vorteilhafterweise ist die Lichtbestrahlungsraumachse und eine senkrechte Standachse der Lichttherapiekammer identisch. Eine Lichtbestrahlungsraumachse verläuft parallel zu einer Längserstreckung der Module bzw. der Panele.

Hierbei können die einzelnen Module zur Bildung einer Gesamtlichtfläche konzipiert sein. Die einzelnen Module schaffen zusammen eine großflächige Lichtabstrahlfläche, sodass die Module zusammenwirken, um innerhalb der Lichttherapiekammer ein (ganz bestimmtes) Licht, z. B. einer (ausgewählten) Lichttemperatur oder einer (ganz bestimmten) Wellenlänge, und eine ganz bestimmte Lichtverteilung hervorrufen zu können. Das Licht strahlt von den einzelnen Modulen ab. Jedes Modul hat seine eigene Strahlrichtung. Die Strahlrichtungen der Module sind allesamt so gerichtet, dass sie auf die Lichtbestrahlungsraumachse deuten. Jede Strahlrichtung ist auf den Zentralbereich der Lichttherapiekammer ausgerichtet. Solche Raumelemente können mit gleichartigen Raumelementen, also von Modul zu Modul, zu einer Lichttherapiekammer zusammengebaut werden.

So ist es möglich, die Lichttherapiekammer aus mehreren gleichartig gestalteten Modulen aufzubauen. Auch hat die Lichttherapiekammer eine Tür. Besonders vorteilhaft ist es, wenn die Tür eine zweiflüglige Tür ist, weil dadurch das Eintreten in die und das Austreten aus der Lichttherapiekammer besonders leicht und komfortabel realisierbar ist. Idealerweise sind die einzelnen Elemente, die als Module Teile der Tür ergeben, gleichartig wie die übrigen Module gestaltet. Insbesondere bedeutet dies, dass jedes Modul eine Bestrahlungsfläche, vorteilhafterweise eine Bestrahlungsgroßfläche hat. Die Türflügel sind somit an einem stationär angeordneten Modul, das das Nachbarmodul zu dem jeweiligen Türflügel ist, angebracht. Natürlich ist es auch möglich, eine nur einflüglige Tür vorzusehen. Eine nur aus einem Flügel bestehende Tür, die somit in der Regel schmaler als eine zweiflüglige Tür ist, kann das einzige bewegliche Modul der Lichttherapiekammer sein. Die übrigen Module, die der Struktur nach der Tür entsprechen, sind als stationär angeordnete Module vorgesehen.

Gleichartige Module enthalten vorzugsweise gleichartige Panele, wie Panele mit einer gleichen Anzahl von Leuchtmitteln. Wenn zwei Module gleichartig sind, haben diese Module insbesondere eine gleiche Höhe und vorzugsweise eine gleiche Breite. Besonders vorteilhaft lassen sich gleichartige Module miteinander kombinieren, die eine gleiche Modulwandstärke haben, wie ein gleicher äußerer Abstand zwischen einer z. B. durch eine Streuscheibe vorgegebenen Ebene und einer dieser gegenüberliegen Ebene, die durch eine Blende gebildet ist.

Werden geeignete Verbindungstechniken eingesetzt, so kann durch die Wahl der entsprechenden Verbindungstechnik aus einem Modul entweder ein stationär anzuordnendes Modul oder ein schwingbares bzw. schwenkbares Modul werden. Die schwingbaren bzw. schwenkbaren Module können die Funktion einer Tür oder eines Türflügels übernehmen. Die stationären Module dienen als Wände der Lichttherapiekammer.

Besonders vorteilhaft ist es, wenn jedes Modul schichtartig, d. h. mit wenigstens einer ersten Schicht und mit wenigstens einer zweiten Schicht und ggf. auch mit weiteren Schichten aufgebaut ist. Dadurch können die Panele durch parallel zueinander verlaufende Schichten realisiert werden.

Die Tür sollte, wie weiter oben schon angesprochen, schwenkbar sein. Hierdurch kann das Innere bzw. der Zentralbereich der Lichttherapiekammer von der Außenwelt getrennt werden. Außenlicht wird abgehalten. Licht des Inneren der Lichttherapiekammer wird vorzugsweise im Inneren der Lichttherapiekammer behalten (daneben können aber natürlich bewusst gestaltete Lichtaustrittskanten und -bereiche, z. B. Lichtkränze, auf bzw. in den Modulen existieren).

Sind die Module gewichtsmäßig, abmessungsmäßig und von ihrer Haptik durchdacht gestaltet, so kann eine Lichttherapiekammer von einer einzigen Person innerhalb von wenigen Arbeitsschritten aufgebaut bzw. zusammengestellt/erstellt werden.

Ein, insbesondere für eine Person handhabbares, Modul sollte länger als breiter sein, sodass ein solches Modul von einer Person aufrecht, vorzugsweise durch ein seitliches Anfassen per Hand, transportiert werden kann. Ein entsprechendes Modul kann elektrisch konfektioniert sein. Die Lichtquellen in einem Modul sind vorteilhafterweise elektrisch zu versorgen.

Über einen entsprechenden Steckverbinder, der vorteilhafterweise an der oberen Schmalseite angeordnet ist, können elektrische Kontaktierungen zwischen Modul und Deckel hergestellt werden. Idealerweise ist also jeweils an einer schmäleren Seite eines Moduls ein Steckverbinder, über den die gleichartig gestalteten Module elektrisch durchkontaktierbar sind.

Eine Gestaltung, bei der die senkrecht aufzustellenden Module mit Steckern an ihrer Oberseite, ausgestattet sind, fördert den Zusammenbau bzw. das Aufstellen einer Lichttherapiekammer. Befindet sich der Stecker an der Seite, die als Oberseite des Moduls vorgesehen ist, so kann nach einem Aufstellen der stationär, d. h. fest anzuordnenden Module abschließend der Deckel aufgelegt werden. Hierdurch kann eine auf oder in dem Deckel sich befindliche Verdrahtungs- bzw. Elektronikbox elektrisch angeschlossen werden.

Die erfindungsgemäße Lichttherapiekammer hat zahlreiche Vorteile. Eine leichte Montage ist eine nicht zu verkennende Erleichterung für einen Monteur.

Die Lichttherapiekammer wird durch mehrere Module, die Teil der Außenhülle sind, und ggf. durch weitere Bauteile, wie z. B. durch einen Deckel oder z. B. durch eine Bodenplatte, geschaffen. Jedes dieser Module hat eine Innenseite, die auf den Zentralbereich der Lichttherapiekammer gerichtet ist. Die Module bilden den Lichtbestrahlungsraum. Der so formierte Lichtbestrahlungsraum hat mehrere Flächen, die zusammen die Gesamtfläche für die Beleuchtung, die Lichtabstrahlfläche, schaffen. Idealerweise ist jede Fläche als Bestrahlungsgroßfläche, insbesondere über die gesamte Höhe eines Moduls sich erstreckend, ausgestaltet. Über die Bestrahlungsgroßfläche gelangt also Licht in den Lichtbestrahlungsraum der Lichttherapiekammer. Obwohl eine in Module aufgeteilte Lichttherapiekammer von möglichst wenigen Personen zusammenzubauen ist, ergibt sich - nach Fertigstellung - eine Bestrahlungsgroßfläche.

Die Lichttherapiekammer sollte so gestaltet sein, dass die einzelnen Module zu einer sich schließenden Hülle zusammengefügt werden können. Durch die Module, ihre Verbindungselemente, wie Standardprofile, und ggf. durch einen Deckel und ggf. durch eine Fundamentplatte wird eine Hülle geschaffen, die den Innenraum vom Äußeren der Lichttherapiekammer trennt. Hierbei kann jedes Modul zusätzlich einen Lichtaustrittskranz aufweisen, durch den z. B. der im Inneren in einem Moment gerade leuchtende Farbton signalisiert wird. So lässt sich jeder Zeit von einer Person die allgemeine Funktionstüchtigkeit der zuvor zusammengestellten Lichttherapiekammer überprüfen (z. B. am Ende des Zusammenbaus).

Die einzelnen Bestrahlungsgroßflächen können durch ihre Flächen zugleich als Reflektor gestaltet sein. Hierdurch ist es möglich, ein von den übrigen Modulen stammendes Streulicht zu reflektieren und wieder auf den Zentralbereich des Lichtbestrahlungsraums zu richten. Durch eine effiziente Nutzung des elektrischen Stroms und durch eine mehrfache Reflektion und Umleitung des Lichts können die Module leichter und kompakter ausgelegt sein, sodass jedes Modul von einer Person alleine getragen werden kann, z. B. während eine Lichttherapiekammer aufgebaut wird.

Die einzelnen Module haben wenigstens eine Fläche, die wie eine Ebene wirkt. Die Fläche ist eben. Diese Ebenen sind idealerweise nach innen gerichtet. Im Inneren der Lichttherapiekammer schließen sich einzelne Ebenen zur Bildung der Lichtabgabeflächen zusammen. Über die Innenseiten der Module bzw. über die Ebenen ist eine mehrfache Umlenkung des eingeleiteten Lichts möglich, insbesondere in dem Fall, dass die Ebenen durch semidurchlässige, reflektierende Flächen gebildet werden. Hinter den Ebenen sind die Leuchtmittel der Lichtquellen angesiedelt. Die Leuchtmittel strahlen durch die Ebenen hindurch. Licht, das im Lichtbestrahlungsraum gelandet ist, wird idealerweise durch die reflektierende Eigenschaft der Ebenen in den Lichtbestrahlungsraum zurückgespiegelt. Ebene Bauteile und Baugruppen, z. B. Module mit ebenen Flächen, erleichtern die Handhabung für eine Person.

Besonders vorteilhaft ist es, wenn weniger als 50 %, vorzugsweise sogar weniger als 20 % des Lichtes, das auf ein Modul aus dem Innenraum einfällt, von einem Modul absorbiert wird. Je größer der reflektierte Anteil ist, desto energiesparender kann die Lichttherapiekammer gestaltet werden, was wiederum zur Förderung der Handhabbarkeit und zur Erleichterung der Montage einen Beitrag leistet.

Auf der rückwärtigen Seite der Module bzw. der Panelen (die nach außen sich zuwendende Seite bzw. die sich nach dem Zusammenbau außen befindliche Seite) ist idealerweise eine äußere Blende angeordnet, die z. B. als schalenartige Aluminiumblende ausgeführt sein kann. Eine solche Aluminiumblende kann an einem Trägerrahmen befestigt werden. Durch Trägerrahmen und Aluminiumblende wird ein Halbgehäuse (ein schalenartiges bzw. einen eine Hälfte des Gehäuses ausmachenden Gehäuseabschnitt) geschaffen, in das die weiteren Bauteile und Baugruppen des Panels bzw. des Moduls eingebracht werden können. Die Module sind für die Zerstörungssicherheit vor leichteren Impulsen ausgelegt, die ein Monteur bei seinem Wuchten der Module (versehentlich) auf die Module ausübt.

Zu den Panelen können eine äußere Blende und/oder ein Trägerrahmen und/oder eine Lichtfläche und/oder ein Berührschutz gehören. Die Lichtfläche und/oder der Berührschutz sind idealerweise als flache Elemente ausgestaltet, die in einer vorteilhaften Weiterbildung zu einem einzigen schichtartigen Bauteil zusammengefügt sind. Auf der äußeren Seite gibt es die äußere Blende. Im Inneren gibt es die Beleuchtungsmittel. Auf der Innenseite des Moduls gibt es den Berührschutz. Eine Berührung der Leuchtmittel ist somit ausgeschlossen. Werden die äußere Blende und der Berührschutz ausreichend mechanisch stabil gestaltet, so ist ein Modul, das von einem ggf. nicht so geschickten Handwerker transportiert wird, auch vor Schlägen und mechanischen Impulsen geschützt.

Die Bestrahlungsgroßfläche wird in der Lichttherapiekammer durch mehrere Bauteile gebildet. So ist es möglich, mehrere reflektierende Streuscheiben nebeneinander anzuordnen, um die Bestrahlungsgroßfläche zu bilden. Ist die reflektierende Streuscheibe mechanisch ausreichend stabil ausgeführt (Kerbschlagzähigkeiten nach Izod oder Charpy von mehr als 4 KJ/m²), so kann sie zugleich einen Berührschutz bilden, der eine Hemmung gegen ein Berühren insbesondere der Leuchtmittel der Lichtquellen darstellt bzw. ein Berühren von Leuchtmittel verhindert. Die Leuchtmittel, die unterhalb der Streuscheibe angeordnet sind, werden durch den Berührschutz, der die Funktion einer Schutzplatte übernimmt, vor einer direkten Berührung, z. B. durch einen Nutzer der Lichttherapiekammer, geschützt. Idealerweise ist somit ein und das gleiche Bauteil sowohl eine reflektierende Streuscheibe, d. h., die Schicht kann zum einen Licht streuen, das auf einer Seite der Scheibe eingeleitet wird, und zum anderen kann die Schicht Licht reflektieren, das auf einer anderen Seite auf die Streuscheibe auftritt, als auch ein Berührschutz. Eine so gestaltete Schutzplatte kann als opake bzw. milchige Platte ausgeführt sein, wodurch Licht gestreut werden kann. Ein geeignetes Material für die Schutzplatte ist ein Polyamid, wobei das Polyamid als schlagfestes Polyamid ausgeführt sein kann. Mechanisch festere Polyamide werden durch kratzfeste Oberflächenbeschichtungen geschaffen. Hierfür kann eine Siliziumoxidschicht oder eine Aluminiumoxidschicht Teil der Oberflächenbeschichtung des Polyamids sein. In die Beschichtung können Kohlenstoffe oder auch nanokristallines Silizium eingearbeitet sein. Somit hat die Polyamid-Schicht eine oberflächige Schicht, die das Polyamid schlagfester macht.

Als Teil der Lichtquellen kommen besonders bevorzugt LED-Streifen (im Sinne von Leuchtmitteln) in Betracht, die auf einem gemeinsamen LED-Träger angeordnet sind. Streifen gibt es als Rollenware, die abgerollt ein längliches, häufig nur wenige Millimeter breites Band darstellen. Solche LED-Streifen können nebeneinander auf dem gemeinsamen LED-Träger aufgebracht sein, z. B. aufgeklebt sein. Idealerweise weist jedes Modul mehrere LED-Streifen auf, von denen mehrere auf einem gemeinsamen LED-Träger aufgebracht sind. Werden die LEDs als LED-Streifen verbaut, sorgt dies für eine größere Unempfindlichkeit der Leuchtmittel (insbesondere mechanische Stabilität).

Die LED-Streifen können sowohl quer als auch senkrecht zum Boden, der die Lichttherapiekammer trägt, ausgerichtet in einem Modul verlaufen. Idealerweise reicht ein LED-Streifen von einer ersten Seite des Moduls bis zu der der ersten Seite gegenüberliegenden Seite. Verlaufen die LED-Streifen in senkrechter Richtung (in Bezug auf den Boden), so erstreckt sich jeder LED-Streifen über die gesamte Lichtabgabehöhe seines Panels. Ist der LED-Streifen quer zur senkrechten Erstreckung des Moduls angeordnet, so erstreckt sich idealerweise dieser LED-Streifen über die gesamte Lichtabgabebreite des Moduls bzw. Panels. Von einer Seite bis zu der anderen Seite der Bestrahlungsgroßfläche erstreckt sich vorteilhafterweise ein einziger LED-Streifen; mehrere LED-Streifen liegen nebeneinander und sind alle gleich lang bzw. breit bzw. hoch.

Dank eines Rahmens können die Module ausreichend stabil ausgeführt werden, sodass jedes Modul ein sich selbst stützendes bzw. frei tragendes Modul ist. Somit kann ein Modul als Montageeinheit verbaut werden. Wird ein Modul auf einer seiner kürzeren Seiten aufgestellt, so kann das Modul senkrecht, d. h. aufgerichtet selbstständig stehen. Entlang der längeren Seite des Moduls kann ein weiteres Modul angeschlossen werden. Eine Befestigung entlang einer Langseite ist möglich. Als Verbindungselement von einem Modul zu einem nächsten Modul stehen Profile zur Verfügung, idealerweise Standardprofile. Durch Befestigung an einem Standardprofil kann links und rechts zu diesem Profil je ein Modul angeordnet werden. Das Standardprofil vermittelt zwischen zwei Modulen; das Standardprofil ist das Verbindungselement zwischen zwei Modulen. Ein solches Standardprofil kann ein Mehrkantprofil sein. Ein Standardprofil stellt das Verbindungsstück zwischen zwei Modulen dar. Das Standardprofil vermittelt zwischen einem ersten Modul und einem zweiten Modul.

Für eine besonders zügige Erstellung einer Lichttherapiekammer, d. h. für einen zügigen Zusammenbau der einzelnen Komponente zu einer Lichttherapiekammer sind die Module alle wenigstens mannshoch. Der Transport der einzelnen Module ist vereinfacht, wenn die Breite eines Moduls nicht breiter als eine übliche Körperbreite ist, z. B. nicht breiter als 80 cm. Dank des schichtartigen Aufbaus kann ein Modul sehr dünn realisiert werden. Vor allem abhängig von der durchströmenden Luft, die für das Kühlen der LED-Streifen benötigt wird, kann eine Wandstärke eines Moduls so gering sein, dass sie nur 2 cm beträgt. Eine Wandstärke von 20 cm ist aber auch gestaltbar, z. B. wenn ausreichende Luftvolumina unterhalb der Blende durchströmen können sollen.

Die Blende des Moduls ist besonders vorteilhaft gegen einen Wärmestau geschützt, indem eine Isolationsschicht unterhalb der Blende vorhanden ist. Eine solche Isolationsschicht kann auch als Schalldämmmatte ausgeführt sein. Das ggf. vorhandene Gebläse, der Ventilator in einem Modul, ist weniger zu hören, wenn eine Schalldämmmatte verbaut ist.

Ein Modul ist besonders leicht und einfach zu transportieren, wenn Maximalgewichte nicht überschritten werden. Arbeitstechnisch günstig ist ein Maximalgewicht von 30 kg. Viele Monteure sind in der Lage, 30 kg alleine zu einem Montageort zu transportieren. Damit ein Modul die Maximalgewichtsgrenze von z. B. 30 kg einhalten kann, können die einzelnen Bauteile des Moduls, wie z. B. die Blende oder wie z. B. der Berührschutz, aus Aluminium und/oder aus Kunststoff gefertigt sein.

Das zuvor angesprochene Standardprofil kann durch ein Mehrkantprofil mit Längsnuten realisiert sein, z. B. durch ein Vierkantprofil mit von oben nach unten entlang des Profils verlaufenden Nuten (bzw. von unten nach oben verlaufenden Nuten). An den Nuten oder in den Nuten können Schraubverbindungen eingebracht sein, durch die die Module mit dem Mehrkantprofil befestigt werden können. Die Nuten können zueinander in einem Winkel angeordnet sein. Mit anderen Worten, die Ausrichtung der Nuten kann so gewählt sein, dass zwischen den Nuten ein Winkel (Innenwinkel) aufgespannt ist. Der Winkel zwischen den Nuten bestimmt die Größe der Lichttherapiekammer. Zwischen solchen Nuten können Winkel von 15°, 30°, 45° oder auch 60° vorliegen. Soll eine viereckige Lichttherapiekammer realisiert werden, so kann der Winkel zwischen den Längsnuten in den Mehrkantprofilen sogar 90° betragen. Über das Standardprofil (bzw. mit Hilfe des Standardprofils) wird also eine Verbindung zu zwei der Module geschaffen.

Wie die Module zueinander angeordnet sind, kann anhand eines Stellwinkels ausgedrückt werden, der auch als Nuteninnenwinkel bezeichnet werden kann. So, wie die Nuten zueinander stehen, so können die Module zueinander ausgerichtet sein. Der Nuteninnenwinkel spiegelt sich in der Ausrichtung der Module wieder. Ein erstes Modul, das stationär platziert ist, und ein zweites Modul, das stationär platziert ist, können durch einen Stellwinkel zueinander ausgerichtet sein. Ein solcher Stellwinkel kann z. B. 45° betragen, wenn die Lichttherapiekammer als Oktagon ausgeführt ist. Es hat sich nämlich gezeigt, dass ein Oktagon eine Form für die Lichttherapiekammer ist, die genug Platz für einen Nutzer im Inneren lässt und zugleich so kompakt ausgeführt ist, dass sie mit Ein-Hand-Modulen bzw. Ein-Mann-Modulen erstellt werden kann.

Die Module stehen vorteilhafterweise auf einer Bodenplatte, die als Fundamentplatte zur Verfügung steht. Die Bodenplatte kann eine wasserdichte Bodenplatte sein, auf der die Module in stehender Weise aufgerichtet sind bzw. auf der sich die Module auf einer Seite ruhend in die Höhe erstrecken.

Gegenüber der Bodenplatte bzw. der Bodenplattform (Fundamentplatte) ist idealerweise der Deckel platziert. Deckel und Fundamentplatte verlaufen in einem solchen Fall parallel zu einander. Die beiden Elemente "Bodenplattform" und "Deckel" sind gegenüberliegend zueinander an zwei unterschiedlichen Enden der Module angeordnet.

Die Lichtausbeute der Lichttherapiekammer kann gesteigert werden, wenn wenigstens eines der Bauteile "Deckel" und "Bodenplattform" eine Lichtabstrahlungsfläche aufweist. In einer besonders günstigen Ausgestaltung haben die Bodenplattform eine Lichtabstrahlungsbodenfläche und der Deckel einen Lichtabstahlungshimmel, wobei letzterer vorzugsweise als Lichtreflektionshimmel ausgebildet ist. Der Himmel strahlt das auf ihn auftreffende Licht wieder zurück.

Der Aufenthalt in der Lichttherapiekammer wird insbesondere in den Wintermonaten und insbesondere für Personen mit Durchblutungsstörungen angenehmer gestaltet, wenn die Bodenplattform eine elektrische Bodenheizung aufweist; diese kann z. B. durch einen Infrarotstrahler realisiert sein.

Zusätzlich kann der Deckel einen Aufsteckbereich aufweisen. Wird der Deckel zur Vervollständigung der Lichttherapiekammer angebracht, ergibt sich - bei einer vorteilhaften Ausgestaltung - eine elektrische Verbindung zu den einzelnen Modulen. Die Module sind über den Deckel kontaktierbar, genauer gesagt über Kontakte, die am Deckel angebracht sind (z. B. Stecker oder Steckverbindungen). Mehrere Module nutzen den Deckel, um über den Deckel elektrische Kontaktierungen herzustellen. Der Deckel stellt nicht nur den oberen Abschluss der Lichttherapiekammer dar, sondern er stellt auch die Steckverbindung zu den Modulen sicher. Steckverbindungen, die möglichst weit oben angesiedelt sind, sind vor mechanischen Schäden geschützt. Sind weibliche Stecker Teil der Module, so ist die Gefahr von Kontaktschäden an den Modulen gering. Werden die Module zunächst senkrecht aufgestellt, um die Lichttherapiekammer, genauer einzelne Wände der Lichttherapiekammer, zu bilden, und wird anschließend der Deckel aufgelegt, so sorgt eine solche Erstellungsweise für eine möglichst geringe Schädigungsgefahr beim Zusammenbau, insbesondere in dem Fall, dass nur eine einzige Person für das Aufstellen der Lichttherapiekammer vorgesehen ist.

Außerdem kann an dem Deckel eine Schließvorrichtung, z. B. eine magnetische Schließvorrichtung, angebracht sein, über die die Tür (bzw. ihre Türflügel) der Lichttherapiekammer in einer verschlossenen Position gehalten wird. Soll die Tür geöffnet werden, ist zunächst eine, wenn auch geringe, Kraft des Magneten der Schließvorrichtung zu überwinden. Die Türen schlagen somit nicht von selbst auf. Die Türen haben eine Vorzugsstellung in der verschlossenen Position. Der Austritt von Licht aus der Therapiekammer wird begrenzt. Wird die Elektronik bzw. die Steuerung der Lichttherapiekammer so gestaltet, dass überprüft wird, ob sich die Tür in der verschlossenen Position befindet, so kann ein Aufleuchten der Lichtquellen der Lichttherapiekammer bis zu dem Zeitpunkt unterbunden bleiben, bis ein sicherer Verschluss der Tür detektiert worden ist. So ist es möglich, die Schließvorrichtung mit einem Türalarm zu koppeln. Auch ist es möglich, die Schließvorrichtung mit einer Zeitschaltuhr zu koppeln. Ist eine gewisse Betriebszeit abgelaufen, so zieht der Magnet einige wenige Minuten gar nicht mehr an. Eine Unterbrechung eines Dauerbetriebs ist zunächst einmal notwendig. Der Betrieb der Lichttherapiekammer kann erst nach einer gewissen Zeitdauer wieder aufgenommen werden. Außerdem ist es möglich, während des Zusammenbaus der Lichttherapiekammer die Tür und ihre Flügel zu arretieren. Der Magnet hält die Tür in der verschlossenen Position. Ein Umkippen von Modulen bei einer leichteren Erschütterung ist ausgeschlossen. Die Tür ist in dem Moment ein statisches Element. Die statisch zu errichtende Kammer, die ortsfest anzusiedelnden Module bleiben an ihrem Ort in senkrechter Errichtung bzw. Aufrichtung stehen.

Wie zuvor schon angesprochen, sind viele Module idealerweise identisch realisiert. In einer besonders günstigen Ausgestaltung rahmen (bzw. flankieren seitlich) zwei zueinander identische Module ein drittes Modul ein. Zwischen den Modulen erstrecken sich die Profile, z. B. Standardprofile, um die Module miteinander zu verbinden.

Steckverbinder können (theoretisch) an nahezu allen Stellen der Module vorgesehen sein. Als besonders vorteilhaft hat es sich erwiesen, an einer der schmäleren Seiten den jeweiligen Steckverbinder für ein Modul zu positionieren. Über die schmäleren Seiten und den Aufsteckbereichen am Deckel können leicht zusammensteckbare elektrische Kontakte erzeugt werden. Idealerweise gibt es keine Kodierung auf den Steckern von einem Modul zu dem nächsten Modul, sondern alle Stecker sind zueinander identisch. In einem solchen Fall ist es egal, mit welchem Modul ein Monteur anfängt, die Lichttherapiekammer zusammenzubauen.

Eines der Module kann zusätzlich mit einem Touchpanel oder einer sonstigen Bedienschnittstelle ausgestattet sein. Ein solches Touchpanel kann sowohl an der Innenwand als auch an der Außenwand des Moduls angebracht sein. Besonders vorteilhaft ist es, wenn eine Bedienschnittstelle auf einer Außenwand eines Moduls angebracht ist, über die z. B. ein Betriebsprogramm, eine Farbwahl und/oder eine Betriebsdauer auszuwählen sind.

Werden Ventilatoren in den Modulen angeordnet, so können die LEDs, die LED-Streifen und die LED-Träger auf einer niedrigeren Temperatur gehalten werden. Eine kompaktere, mit einer höheren Lichtdichte ausgestattete Bestrahlungsgroßfläche kann realisiert werden. Dadurch können die Module kompakter gehalten werden. Je kompakter die Module sind, z. B. je flacher die Wand eines Moduls realisiert ist, desto einfacher ist die Montage, insbesondere in dem Fall, dass nur eine einzige Person die Lichttherapiekammer aufstellen soll.

Der Ventilator kann an einer Modulschmalseite, z. B. unten oder oben im Modul, angeordnet sein. Über den Ventilator kann eine Luftströmrichtung durch das Modul bestimmt werden. Mit Hilfe des Ventilators kann Luft von einer ersten Modulschmalseite zu einer zweiten Modulschmalseite bewegt werden. Die Luftdurchströmung kann so z. B. von unten nach oben entlang der Modulwand (insbesondere im Inneren der Modulwand) erfolgen.

Eine Lichttherapiekammer mit einem Zentralbereich lässt ein elektrisch erzeugtes Licht auf diesen Zentralbereich in ihrem Inneren leuchten, wobei sich die Lichttherapiekammer aus als Module gestalteten Panelen und einem Deckel zu einer Zelle zusammensetzt. Die nebeneinander angeordneten Panele bilden ein Vieleck. Im Inneren kann eine Person entlang einer im Zentralbereich verlaufenden Lichtbestrahlungsraumachse zumindest stehen. Durch den modulartigen Aufbau wird ein unkompliziertes und damit flexibles Auf- und Abbauen der Lichttherapiekammer ermöglicht, sodass z. B. eine einzige Person selbstständig einen Transport und eine Montage der Lichttherapiekammer durchführen kann.

Eine Lichttherapiekammer, beispielsweise als verschließbare Zelle, die mindestens das Eintreten und Aufhalten eines Menschen in ihr möglich macht, ist mit einer ersten elektronischen Steuerungs- und Überwachungseinheit ausgestattet. Die Steuerungs- und Überwachungseinheit steht mit Lichtquellen der Lichttherapiekammer in elektronischer Verbindung. So kann die Steuerungs- und Überwachungseinheit zum Ein- und Ausschalten und zur Funktionsüberwachung der Lichttherapiekammer genutzt werden. Eine zweite Steuereinheit kann über eine Internetverbindung die von der ersten Steuerungs- und Überwachungseinheit erfassten (Mess-)Daten empfangen und speichern. Die zweite Steuereinheit kann außerdem in die Funktion der ersten Steuerungs- und Überwachungseinheit, also in den Betriebszustand der Lichttherapiekammer eingreifen. So kann aus der Ferne festgestellt werden, dass die Lichttherapiekammer in einem unerwünschten, die Sicherheit des Nutzers möglicherweise gefährdenden Betriebszustand ist.

Eine besonders vorteilhafte Ausgestaltung der zuvor vorgestellten Lichttherapiekammer umfasst die folgenden markanten Aspekte:
Eine Lichttherapiekammer hat, wenn ihre Bauform auf der Form eines Achtecks ("Oktagon") fußt, eine sehr gute Ausleuchtung an jeder Stelle im Raum. Somit hat die Abwendung von den zuvor bekannten Sonnenbank ähnlichen Geräten nicht nur den Transport der insgesamt recht voluminösen Geräte gelöst, sondern auch noch die positiven Effekte für einen Nutzer bzw. Anwender gesteigert. Aufgrund des Aufliegens des Nutzers auf einer der beiden Flächen in einer Sonnenbank ist eine lichttherapeutische Bestrahlung nicht so gleichmäßig wie bei einem Lebewesen, das in dem Lichttherapierraum bzw. in der Lichttherapiekammer steht oder sitzt.

Aufgrund der vielen LEDs, z. B. als RGB-LEDs realisiert, sind hohe Helligkeiten der unterschiedlichen Farbtöne möglich. Werden RGB-LEDs verwendet, ist jede LED in der Lage, Rot, Grün, Blau und entsprechende Farbmischungen abzustrahlen. Wird in einer als Oktagon realisierten Lichttherapiekammer mit einem Luxmeter die Helligkeit bzw. die Beleuchtungsstärke an einem beliebigen Punkt im Innenraum gemessen, so lassen sich die folgenden Beleuchtungsstärken feststellen, wenn von den Panelen ausschließlich jeweils die Wellenlänge(n) im Bereich des angegebenen Farbtons abgestrahlt wird/werden:
- bei Grün ca. 125.000 Lux,
- bei Rot ca. 50.000 Lux,
- bei Weiß (durch Farbaddition mehrerer Farbspektren) ca. 200.000 Lux und
- bei Blau ca. 40.000 Lux.

Eine solche Helligkeit bzw. Beleuchtungsstärke in einer achteckigen Zelle kann z. B. von 34.656 LEDs erzeugt werden. In einem Panel von acht, die Lichttherapiekammer bildenden Panelen können hierbei z. B. bis zu 4.332 LEDs angeordnet sein, die auf 12 Streifen mit in Serie verschalteten 361 LEDs aufgeteilt sind.

In einer alternativen Ausführungsform sind auf 7 Innenseiten (eines Heptagons), jede Teil einer äußeren Wand, insgesamt 35.000 LEDs platziert. Jedes Panel hat 5.000 LEDs, wobei 250 LEDs zu einem Streifen gehören.

Befinden sich in einem Panel LEDs (den so genannten Hochleistungs-LEDs) mit höherer Helligkeit (größerem Lichtstrom), so kann es in einer alternativen Ausführungsform auch schon ausreichen, dass nur 7.000 LEDs alle Lichtquellen für die Beleuchtung des Innenraums bilden.

Bei dem so gestalteten Lichttherapieraum handelt es sich um eine Zelle mit acht Wandsegmenten und einem Deckensegment bzw. Deckelsegment. Diese Teile können zusammengesetzt werden, um eine Lichttherapiekammer aufzubauen. Die einzelnen Wandmodule der Lichttherapiekammer haben eine Höhe von zwei Metern und können aufgrund ihrer Länge und ihrer Breite (von 80 cm) in einem handelsüblichen Kombinations-Kraftwagen transportiert werden.

Der Innenraum zwischen den Wandmodulen hat einen solchen Durchmesser, dass darin auch eine Person auf einem Stuhl, z. B. auf einem im Design auf die Kammer angepassten Plexiglasstuhl, sitzen kann (die Fläche in der Lichttherapiekammer ist ein Achteck mit einer lichten Breite von 82 cm). Die Lichttherapiekammer ist also aus acht Wandmodulen aufzubauen, von denen zwei Module jeweils mit Scharnieren mit den Nachbarmodulen verbunden sind und die anderen Module über Steckverbinder verbunden sind. Bis auf die Abweichungen zwischen den Steckverbindern, die bei sechs Modulen gleich sind, und den Scharnieren, die bei zwei Modulen gleich sind, sind alle acht Module zueinander identisch aufgebaut. Unter Vernachlässigung der individuellen Verbindungstechnik kann daher davon gesprochen werden, dass die Module identisch aufgebaut sind. Das Gewicht von einem Modul ist vorteilhafterweise derart begrenzt (weniger als 30 kg, vorteilhafterweise sogar weniger als 25 kg), dass das Modul von einer Person (idealerweise alleine) getragen werden kann.

Idealerweise ist der Aufbau jedes einzelnen Moduls im Inneren immer gleich. Ein Modul wird auf seiner Innenwandseite durch eine Plexiglaswand (satiniertes Plexiglas) abgeschlossen bzw. begrenzt, die als Streuscheibe ausgebildet ist. Eine so gestaltete Plexiglasscheibe wird durch Profile gehalten. Hinter der Plexiglasscheibe befinden sich dicht aneinandergereiht LED-Streifen (LED-Stripes) zu je 4.332 LEDs. Die LED-Stripes verlaufen senkrecht, d. h. jeder LED-(Teil-)Streifen erstreckt sich vom Boden zur Decke der Lichttherapiekammer. Ein erster LED-(Teil-)Streifen ist mit einem zweiten LED-Teil-Streifen elektrisch in Reihe verbunden. Die einzelnen LED-(Teil-)Streifen sind zu einem LED-Streifen verbunden. Hinter den LED-Streifen befindet sich eine Trägerplatte. Die Versorgungselektronik sowie die elektrische Zuleitung, die in Kühlkanälen bzw. großflächig zu kühlenden Innenwänden sitzen, sind auf der Rückseite, vorzugsweise möglichst weit oben (vom Boden entfernt), der Trägerplatte angeordnet. Jedes Modul hat drei Standard-Netzteile als Versorgungselektronik. Durch die Kühlkanäle wird Luft, durch ein Gebläse beschleunigt, geleitet. Die elektrische Leistungsaufnahme aller verwendeter Netzteile (8 Module mit je drei Netzteilen, also 24 Netzteile) liegt bei etwa sechs Kilowatt. Ein Gerät hat insgesamt 16 Öffnungen für Luft (8 für einen Einlass und 8 für einen Auslass der Luft). In dem Deckel bzw. auf dem Deckel in einem eigenen Gehäuse als Teil des Deckels ist die Steuerungselektronik für die Ansteuerung der LEDs und für den zu wählenden Lichtfarbton angeordnet. Das satinierte Plexiglas schluckt nur ca. 8 % des Lichts und reflektiert Streulicht.

Durch Licht können verschiedene positive medizinische bzw. körpereigene Effekte erzeugt werden:
1. Rotes Licht fördert die menscheigene Kollagen-Produktion.
2. Blaues Licht kann zur Vernichtung von Bakterien, z. B. bei Akne oder Neurodermitis, genutzt werden.
3. Mit grünem Licht kann die Hautpigmentierung gefördert werden.
4. Oranges Licht hat positive Effekte auf Haarwachstum, z. B. bei Teilglatzen.
5. Gelbes Licht kann als Hilfsmittel zur Aufhellung der Haut genutzt werden.

Eine solche Lichttherapiekammer, die auch als "Farbtherapieraum" bezeichnet werden kann, kann an ein Solarium vermietet oder verkauft werden. Solarien haben die Schwierigkeit, dass sie Grenzwerte für UV-A-Strahlung und UV-B-Strahlung beachten müssen. In solchen Einrichtungen, wie z. B. kommerziell betriebenen Sonnenstudios, kann ein Farbtherapieraum als mögliche Alternative oder als Ergänzung für klassische Sonnenbänke Besuchern angeboten werden. Besucher eines Solariums lassen sich eine Zeit lang mit UV-Strahlen bestrahlen und unterziehen sich einer Farbtherapie zur Abrundung ihres Aufenthalts.

Eines oder mehrere der nachfolgend angesprochen Komponenten und Module können Teile einer Steuerungselektronik sein, die vorteilhafterweise auf dem Dach eines Geräts angeordnet ist:
Teile für eine solche Steuerungselektronik können ein GPS-Sensor, ein LTE-Modul bzw. ein GPRS-Modul, eine WLAN-Anbindung, eine Bluetooth-Anbindung, ein Touch-Screen und eine Anbindung an einen zentral platzierten Server sein.

Eine Betriebsdauerphase für eine Person kann von dieser zwischen 10 Minuten, 15 Minuten und 30 Minuten ausgewählt werden.

Über einen Touch-Screen kann die Farbtherapie ausgewählt werden.

In einer besonders interessanten Weiterbildung kann über ein an der Oberfläche angebrachtes Touch-Screen Musik für eine Beschallung in der Kammer ausgewählt werden.

Je nach zu behandelnden Symptomen ist der richtige Farbton und die richtige Dauer auszuwählen. Bakterienabtötung erfolgt zum Teil schon nach einer 10 Minutenbehandlung mit Blaulicht. Anti-Aging der Haut benötigt eine längere Behandlung, z. B. eine Bestrahlung von 30 Minuten Dauer mit einem Licht einer roten Wellenlänge.

Nach der Behandlung in Farblichttherapiekammern konnten bei vielen (Test-)Personen positive Effekte, insbesondere an der Haut sowie bei deren Allgemeinbefinden, festgestellt werden. Teilweise reichte es, dass eine (Test-)Person die Kammer nur ein einziges Mal aufgesucht hatte.

Die zuvor dargestellten Kombinationen und Ausführungsbeispiele lassen sich auch in zahlreichen weiteren Verbindungen und Kombinationen betrachten.

Soll die Lichttherapiekammer größer ausgestaltet sein, z. B. eine größere Grundfläche umschließen, um z. B. zwei Personen gleichzeitig aufnehmen zu können, so können Profile mit einem kleineren Stellwinkel verwendet werden. Werden Profile verwendet, die einen Stellwinkel von 30° haben, so ergibt sich eine Lichttherapiekammer, die als Dodekagon mit gleichen Seiten ausgeführt ist. Bei einem Oktagon haben alle Profile vorteilhafterweise einen gleichen Winkel von 45°.

Wird die Lichttherapiekammer zusätzlich mit UV-LEDs ausgestattet, so kann einem Nutzer eine Solariumsbehandlung und eine Lichttherapie in der gleichen Kammer angeboten werden. Wird die UV-Bestrahlung gemessen oder berechnet, so kann der Nutzer unterhalb einer maximalen Dosis mit UV bestrahlt werden und eine restliche Nutzungsdauer kann durch eine Lichttherapie ausgefüllt sein.

### Figurenkurzbeschreibung

Die vorliegende Erfindung kann noch besser verstanden werden, wenn Bezug auf die beiliegenden Figuren genommen wird, die beispielhaft besonders vorteilhafte Ausgestaltungsmöglichkeiten darlegen, ohne die vorliegende Erfindung auf diese einzuschränken, wobei
Figur 1 in vereinfachter, schematischer Darstellung eine erste Ausführungsform einer Lichttherapiekammer in Explosionsdarstellung zeigt,
Figur 2 eine Ansicht von oben einer in Komponenten zerlegten Lichttherapiekammer nach Figur 1 zeigt,
Figur 3 ein Modul einer weiteren Ausführungsform in Explosionsdarstellung zeigt,
Figur 4 eine weitere Ausführungsform einer Lichttherapiekammer mit geöffneten Türen zeigt,
Figur 5 die Lichttherapiekammer nach Figur 4 mit geschlossenen Türen zeigt,
Figur 6 eine weitere Ausführungsform einer Lichttherapiekammer in einer Ansicht von oben zeigt,
Figur 7 eine weitere Ausführungsform einer Lichttherapiekammer in Verbindung mit einer Fernüberwachungsdatenverbindung zeigt und
Figur 8 eine weitere Ausführungsform eines Moduls in Explosionsdarstellung zeigt, das so gestaltet ist, mit weiteren Modulen, wie dem Modul nach Figur 3, zu einer Lichttherapiekammer kombiniert zu werden.

### Figurenbeschreibung

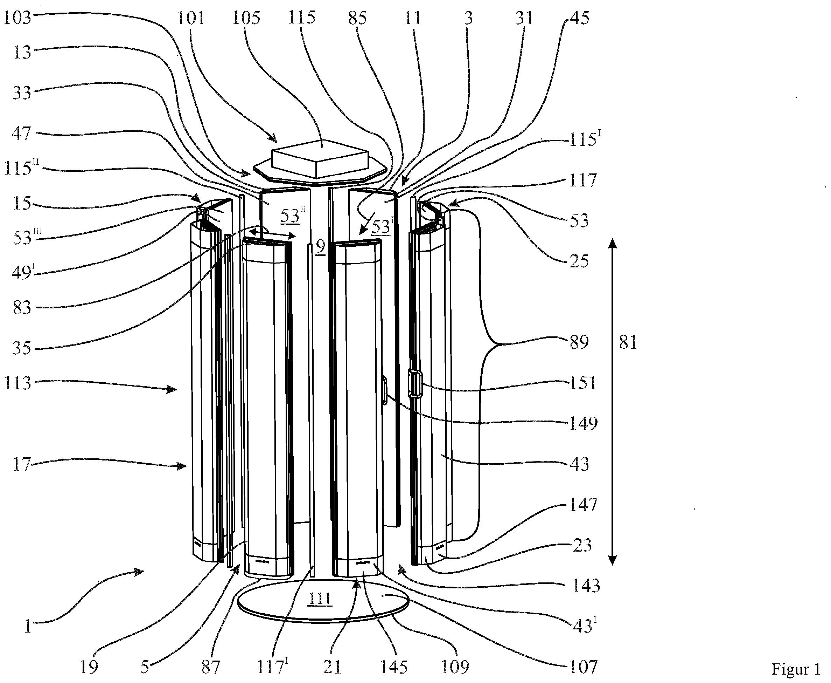

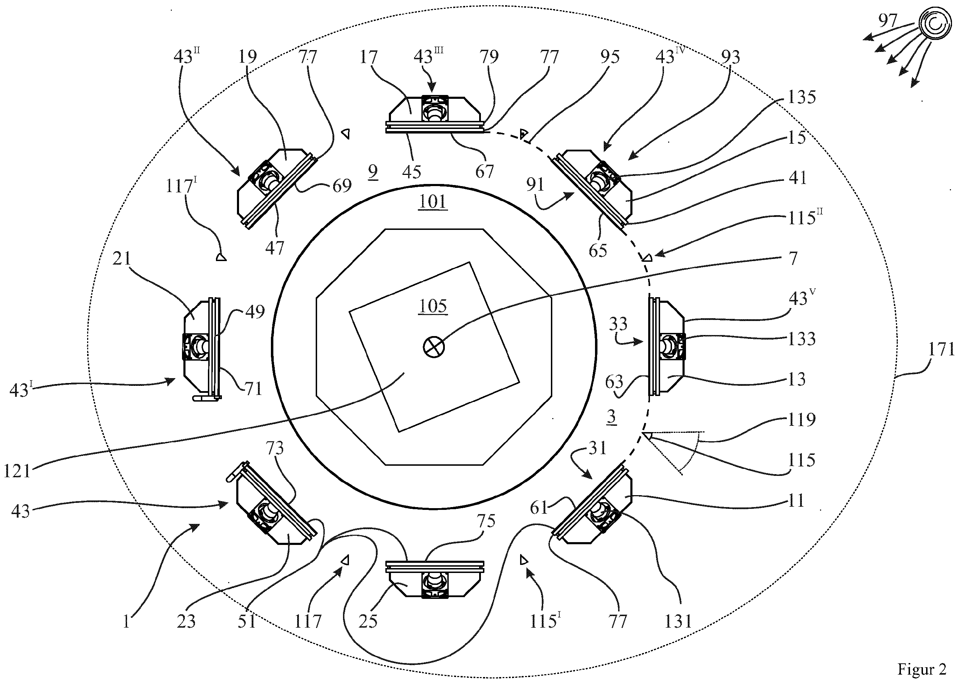

Die Lichttherapiekammer 1, die in den Figuren 1 und 2 in Explosionsdarstellungen aus zwei verschiedenen Perspektiven gezeigt ist, hat in ihrem Inneren 9 einen Lichtbestrahlungsraum 3, der den Zentralbereich 5 der Lichttherapiekammer 1 umfasst. Durch diesen Zentralbereich 5 verläuft, wie besonders gut anhand von Figur 2 zu sehen ist, eine Lichtbestrahlungsraumachse 7. Die Lichtbestrahlungsraumachse 7 durchsetzt den Lichtbestrahlungsraum 3.

Die in den Figuren Figur 1 und Figur 2 gezeigten, einzelnen Module 11, 13, 15, 17, 19, 21, 23, 25, d. h. Module, die zu einem Vieleck 113, genauer gesagt zu einem Achteck zusammenzufügen sind (siehe Figur 1), haben einen panelartigen Charakter, dies aufgrund ihrer Panelstruktur, was anhand eines ersten Panels 31 und eines zweiten Panels 33 sowie eines weiteren Panels 35 gezeigt ist. Einzelne Panels, wie das erste Panel 31 und das zweite Panel 33, werden über Standardprofile 115 miteinander verbunden. Neben den Standardprofilen 115, 115^{I}, 115^{II} eines ersten Typs hat die Lichttherapiekammer 1 ein Standardprofil eines zweiten Typs 117, 117^{I}. Die Standardprofile 115, 115^{I}, 115^{II} des ersten Typs und die Standardprofile 117, 117^{I} des zweiten Typs ähneln sich weitgehend. Standardprofile 115, 115^{I}, 115^{II} des ersten Typs und Standardprofile 117, 117^{I} des zweiten Typs unterscheiden sich in Bezug auf die Verbindungstechnik zu den Modulen 11, 13, 15, 17, 19, 21, 23, 25. An den Standardprofilen 117, 117^{I} des zweiten Typs sind Scharniere "angeschlagen" (eigentlich: "befestigt"), damit eines der durch die Standardprofile 117, 117^{I} des zweiten Typs verbundenen Module 19, 21 bzw. 23, 25 schwenkbar (im Sinne einer verschließbaren Tür 143) sind. Werden die Module 11, 13, 15, 17, 19, 21, 23, 25 über ihre Profile 115, 115^{I}, 115^{II}, 117, 117^{I} miteinander verbunden, so ergibt sich, wenn der Deckel 101 von oben auf die Module 11, 13, 15, 17, 19, 21, 23, 25 aufgesetzt ist, eine geschlossene Zelle 171 (siehe Figur 2). Aufgrund des Stellwinkels 119 (siehe Figur 2), der durch ein Profil 115 bestimmt wird, genauer wie die Module 11, 13 zueinander an dem Profil 115 befestigt werden können, ergibt sich die Ausrichtung des einen Moduls 11 zu dem anderen Modul 13. Je nach Stellwinkel 119, der bei der Lichttherapiekammer 1 (gem. Figur 1) 45° beträgt, ergibt es sich, dass acht Module 11, 13, 15, 17, 19, 21, 23, 25 benötigt werden, um wieder an das letzte Modul 25 mit dem ersten Modul 11 zu gelangen und eine in einer Ebene geschlossene Hülle durch die Module 11, 13, 15, 17, 19, 21, 23, 25 zusammengestellt zu haben.

Die lichtabstrahlenden Seiten, die (in Figur 2 gezeigten) Bestrahlungsgroßflächen 61, 63, 65, 67, 69, 71, 73, 75 sind nach innen zum Lichtbestrahlungsraum 3 gerichtet. Die Bestrahlungsgroßflächen 61, 63, 65, 67, 69, 71, 73, 75 schaffen zusammen die Lichtabstrahlfläche 51, die sich somit über alle Module 11, 13, 15, 17, 19, 21, 23, 25 erstreckt.

Die Lichttherapiekammer 1, wie es anhand von Figur 2 zu sehen ist, hat Module 11, 13, 15, 17, 19, 21, 23, 25, die zu der Innenseite hin ebenenartig durch ihre jeweiligen Ebenen 45, 47, 49 gestaltet sind. Diese Ebenen 45, 47, 49 stellen eine erste Schicht 77 der Module 11, 13, 15, 17, 19, 21, 23, 25 dar. Jedes der Module 11, 13, 15, 17, 19, 21, 23, 25 ist mehrschichtig aufgebaut und hat somit neben einer ersten Schicht 77 eine zweite Schicht 79. Durch das schichtartige Anordnen der einzelnen Baugruppen und Funktionselemente der Module 11, 13, 15, 17, 19, 21, 23, 25 lässt sich ein flaches Modul 11, 13, 15, 17, 19, 21, 23, 25 herstellen, für das nur eine einzige Person benötigt wird, um es zu handhaben.

Besonders gut ist in Figur 1 die Lichtabgabehöhe 81 und die Lichtabgabebreite 83 der einzelnen Panelen 31, 33, 35 zu sehen. Wie sich aus der Figur 1 ergibt, sind die Module 11, 13, 15, 17, 19, 21, 23, 25 aufgrund ihrer Abmessungen und ihres Gewichts von einer Person handhabbar. Eine breitere Seite 89 eines Moduls 23 entspricht im Wesentlichen einer Lichtabgabehöhe 81 des Moduls 23. Eine schmälere Seite 87 eines Moduls 19 entspricht im Wesentlichen einer Lichtabgabebreite 83 des Moduls 19.

Die, in Figur 2 zu sehende, geschlossene Zelle 171 sperrt Außenlicht 97 durch ihre Hülle 95 ab, die durch ein Zusammenfügen der Module 11, 13, 15, 17, 19, 21, 23, 25 entsteht. Von außen ist die auf jedem der Module 11, 13, 15, 17, 19, 21, 23, 25 vorhandene Blende 43, 43^{I}, 43^{II}, 43^{III}, 43^{IV} zu sehen, die die Außenseite 93 bildet. Jede der Blenden 43, 43^{I}, 43^{II}, 43^{III}, 43^{IV}, 43^{V} ist eine Aluminium-Blende, die das Außenlicht 97 nicht auf die Innenseite 91 durchtreten lässt. Die Blende 43^{IV} schließt sich an den Trägerrahmen 41 an. Hinter den Blenden 43^{IV}, 43^{V} sitzen Lüfter, wie z. B. ein erster Lüfter 131, ein zweiter Lüfter 133 und ein dritter Lüfter 135. Der Betrieb der Lüfter 131, 133, 135 wird über die Elektronikbox 105, die auf dem Deckel 101 angeordnet ist, gesteuert bzw. temperaturabhängig geregelt.

In einer Zusammenschau der Figuren Figur 1 und Figur 2 ergibt sich, dass die auf die Innenseite 93 der Module 11, 13, 15, 17, 19, 21, 23, 25 ausgerichteten Lichtquellen 53, 53^{I}, 53^{II}, 53^{III} (siehe Figur 1) unterhalb der ersten Ebene 45 für eine Strahlrichtung 85 (siehe Figur 1) in das Innere 9 der Lichttherapiekammer 1 des speziell für eine Lichttherapie erzeugten Lichts zuständig sind. Durch die erste Ebene 45 und die zweite Ebene 47 der Module 11, 13, 15, 17, 19, 21, 23, 25 lässt sich Licht im Inneren 9 der Lichttherapiekammer 1 reflektieren und umlenken. Durch den Lichtabstrahlungshimmel 103 (siehe Figur 1), der Teil des Deckels 101 mit der Elektronikbox 105 ist, wird der Wirkungsgrad im Inneren 9 weiter gesteigert. Außerdem ist die Bodenplatte 107 nicht nur eine Fundamentplatte 109, sondern sie wirkt als Lichtabstrahlungsbodenplatte 111. Die Tür 143, die durch ein Schwenken der beiden Türflügel 145, 147, insbesondere durch ein Angreifen der Türgriffe 149, 151, verschlossen werden kann, wird von der Elektronikbox 105 überwacht.

In der Elektronikbox 105 ist die erste Steuerungs- und Überwachungseinheit 121 (siehe Figur 2) eingebaut, die u. a. überwacht, ob sich die Tür 143, genauer ob sich ihre Türflügel 145, 147 in der verschlossenen Position befinden (durch nicht einsehbare Bimetall-Magnet-Schalter, die auch als "Tür-Interlock" bezeichnet werden können). Außerdem überwacht die Steuerungs- und Überwachungseinheit 121 die Temperatur hinter den Blenden 43, 43^{I}, 43^{II}, 43^{III}, 43^{IV}, 43^{V} und somit (indirekt) die Temperatur der Lichtquellen 53, 53^{I}, 53^{II}, 53^{III}. Die Steuerungs- und Überwachungseinheit 121 überwacht zum einen die Temperatur der Lichtquellen 53, 53^{I}, 53^{II}, 53^{III} und stellt zugleich (zum anderen) einen Zusammenhang mit den Betriebszeiten der Lichtquellen 53, 53^{I}, 53^{II}, 53^{III} her. Hierdurch ist die Steuerungs- und Überwachungseinheit 121 in der Lage, Restlebensdauern für die Lichtquellen 53, 53^{I}, 53^{II}, 53^{III} permanent mitzurechnen. Wird während des Betriebs einer der Türflügel 145,147 geöffnet, so schaltet die Steuerungs-und Überwachungseinheit 121 die Lichtquellen 53, 53^{I}, 53^{II}, 53^{III} aus. Eine Gefahr der Bestrahlung mit hohen Lichtdosen für Personen vor der Lichttherapiekammer 1 ist damit unterbunden.

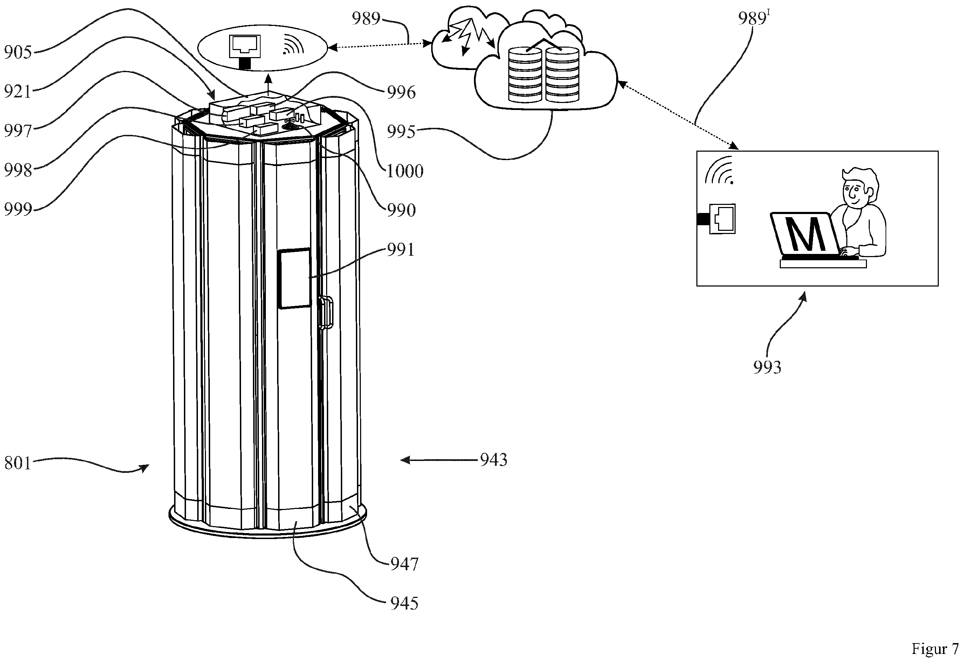

Vorteilhafterweise hat die Steuerungs- und Überwachungseinheit 121 weitere Überwachungsschaltkreise integriert (Leuchtdichtemessung, Betriebszeitpunktarchivierung, Farbprogramme, Helligkeitsmessungen usw.), sodass die Funktionstüchtigkeit der Lichttherapiekammer 1 durch mehrere Messungen überwacht werden kann und ggf. die Lichttherapiekammer 1 auch ganz abgeschaltet werden kann. Fällt eine der Bestrahlungsgroßflächen 61, 63, 65, 67, 69, 71, 73, 75 (vgl. Figur 2) aus, so sinkt die Helligkeit im Inneren 9 der Lichttherapiekammer 1 und die Steuerungs- und Überwachungseinheit 121 kann einen gestörten Betriebszustand über ihr integriertes LTE-Telefongerät bzw. LTE-Datenmodul an eine (in Figur 1 nicht dargestellte) Zentralrechnereinheit (vgl. auch Figur 7 mit dem Zentralrechner 995) melden.

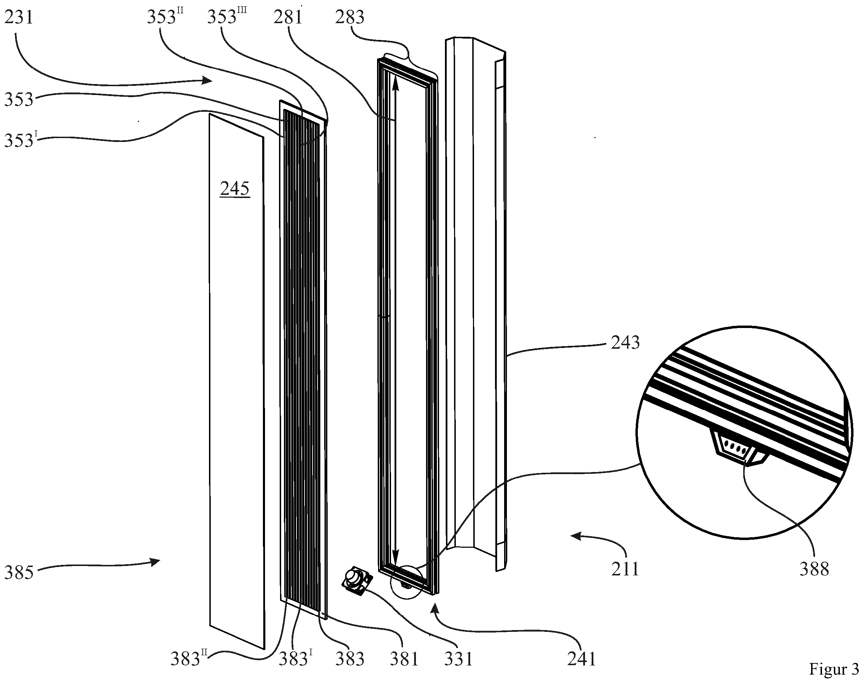

Figur 3 zeigt ein Panel 231 bzw. ein erstes Modul 211 in einer Explosionsdarstellung. Das Modul 211 ist auf einer der Seiten, die als äußere Seite gedacht ist, mit einer Aluminiumblende 243, die schalenartig ausgestaltet ist, abgedeckt, d. h. rückwärtig verschlossen. Das zentrale, mechanisch tragende Bauteil für das Panel 231 ist der Trägerrahmen 241, der ein länglicher, höher als breiter gestalteter Rahmen ist. Durch den Trägerrahmen 241, genauer durch die Innenabmessungen des Trägerrahmens 241 bestimmt sich der zur Verfügung stehende Platz für LED-Streifen 383, 383^{I}, 383^{II} und somit für die Lichtabgabebreite 283 und die Lichtabgabehöhe 281. In den Trägerrahmen 241 kann der LED-Träger 381 eingelegt werden. Der LED-Träger 381 ist das Unterstützungsbrett bzw. die Unterstützungsstruktur für die LED-Streifen 383, 383^{I}, 383^{II}. Die LED-Streifen 383, 383^{I}, 383^{II} haben eine Trägerstruktur, die Leiterbahnen umfasst bzw. in Gestalt einer Platine mit Leiterbahnen und elektronischen Bauteilen für den Betrieb der LEDs auf dem LED-Streifen 383, 383^{I}, 383^{II} ausgeführt ist. Die mechanische Stabilität für die LED-Streifen 383, 383^{I}, 383^{II} und damit für die Leuchtmittel 353, 353^{I}, 353^{II}, 353^{III} wird vorrangig durch den LED-Träger 381 geboten. Die elektrische Leitfähigkeit zwischen den LEDs eines LED-Streifens 383, 383^{I}, 383^{II} ist durch die Trägerstruktur des LED-Streifens 383, 383^{I}, 383^{II} sichergestellt. Die LED-Streifen 383, 383^{I}, 383^{II} sind durch die reflektierende Streuscheibe 385, die zugleich einen Berührschutz darstellt, abgedeckt. Diese Fläche der Streuscheibe 385 spannt eine Ebene 245 auf, durch die das Panel 231 innwandig eine gleichmäßige, für eine Lichtabgabe bestimmte Fläche erhält. Zwischen dem LED-Träger 381 und der schalenartigen Blende 243 ist ein Lüfter (Ventilator 331) für Luftkonvektion durch das Innere des Panels 231 vorhanden. Die Luft wird durch den Ventilator 331 angezogen und hinter der Blende 243 an der oberen Kante des Panels 231 ausströmen gelassen. Die Luft unterströmt den LED-Träger 381 und kühlt somit die LED-Streifen 383, 383^{I}, 383^{II}. Der Lüfter 331 bzw. der Ventilator 331 ist somit das Kühlmittel für sein Panel 231. Aus mehreren solchen Ebenen 245 kann die Innenseite eines Lichttherapieraums, z. B. eines Lichttherapieraums 1 nach Figur 1, zusammengesetzt werden. Die reflektierende Streuscheibe 385 ist aus einem schlagfesten Polyamid mit einer kratzfesten Oberflächenbeschichtung hergestellt. Am unteren Rand des Trägerrahmens 241 sitzt ein Stecker 388, der für eine elektrische Verbindung zwischen Elektronikbox (siehe z. B. die Elektronikbox 105 nach Figur 1) und den LED-Streifen 383, 383^{I}, 383^{II} sowie zu dem Lüfter 331 sorgt. Das Modul 211 nach Figur 3 ist für eine Lichttherapiekammer gestaltet, deren elektronische Steuerung (zumindest die erste Steuereinheit) im Bodenbereich der Lichttherapiekammer sitzt.

Das Modul 211 wiegt nicht mehr als 25 Kilogramm. Es hat eine Breite von 80 cm und eine Höhe von weniger als 250 cm. Das Modul 211 kann somit von einer Person transportiert, gehalten und auch mit anderen, gleichartigen Modulen montiert werden.

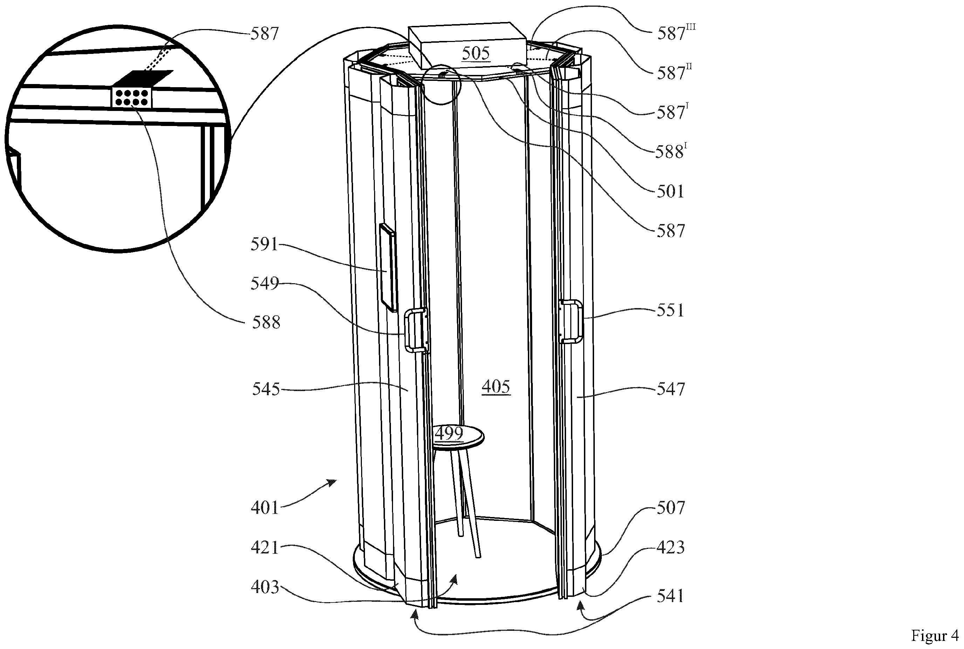

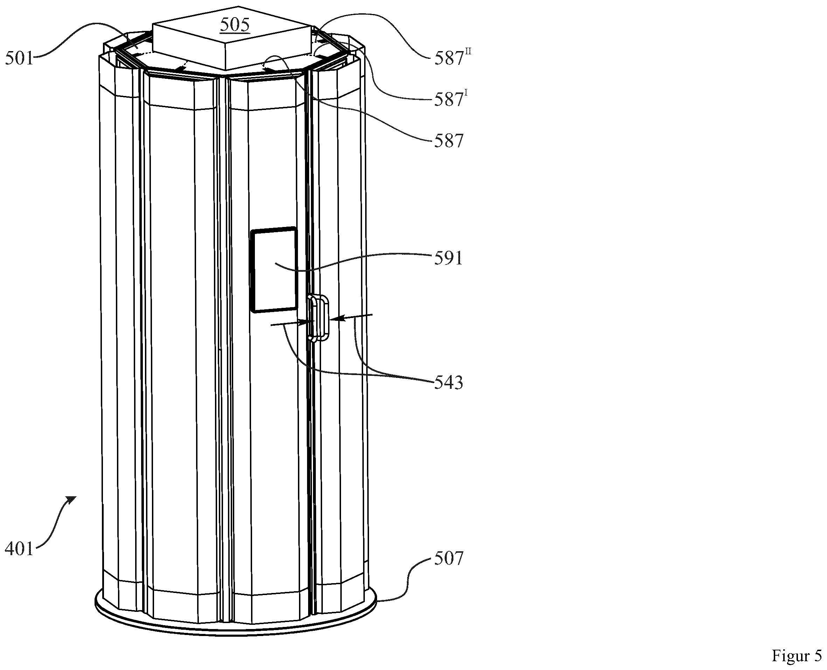

Weitere Details lassen sich anhand des Ausführungsbeispiels einer Lichttherapiekammer 401 nach den Figuren 4 und 5 besser erkennen, die gleichermaßen auch in einer zusammengebauten Lichttherapiekammer 1 gem. den Figuren 1 und 2 vorzufinden sind. Die Figuren 4 und 5 stellen die Lichttherapiekammer 401 mit offener Tür 541 (siehe Figur 4) und mit geschlossener Tür 543 (siehe Figur 5) dar.

Wie aus Figur 4 ersichtlich (dank der offenen Tür 541 zu sehen ist), ist die Lichttherapiekammer 401 so hoch, dass nicht nur ein Stuhl 499 in den Lichtbestrahlungsraum 403 gestellt werden kann, sondern hierauf auch eine (nicht eingezeichnete) Person sitzen kann. Dadurch kann sich eine Person im Zentralbereich 405 des Lichtbestrahlungsraums 403 aufhalten. Der Stuhl 499 steht dabei auf der Bodenplatte 507.

Auf einem der Türflügel 545, 547 mit den Türgriffen 549, 551 sitzt das Bedienfeld 591, ein Touch-Panel. Über das Touch-Panel 591 kann ein Nutzer der Elektronikbox 505 der Lichttherapiekammer 401, wobei die Elektronikbox 505 auf dem Deckel 501 der Lichttherapiekammer 401 montiert ist, ein Farbprogramm und eine Betriebszeit bzw. eine Betriebsdauer vorgeben. Elektrische Signale an die Module 421, 423 werden über die elektronischen Verbindungen 587, 587^{I}, 587^{II}, 587^{III} von der Elektronikbox 505 weitegeleitet. Zur Weiterleitung der elektronischen Signale gibt es einen Stecker 588, 588^{I} hinter jedem Modul 421, 423, also auch hinter den Türflügeln 545, 547. Die elektronischen Verbindungen 587, 587^{I}, 587^{II}, 587^{III} sind in dem Deckel 501 zu der Elektronikbox 505 geführt. Auch die Stecker 588, 588^{I} sind in dem Deckel 501 eingelassen. Bei einem Zusammenbau der Lichttherapiekammer 401 sind die empfindlicheren elektronischen Bauteile und Leiterbahnen sowie die Steckverbindungen wie die Stecker 588, 588^{I} vor Beschädigungen durch Impulse, Schläge und Erschütterungen geschützt. Dies erleichtert den Zusammenbau einer Lichttherapiekammer 1 an einem beliebigen Ort, z. B. in einem Sonnenstudio während Publikumsverkehr.

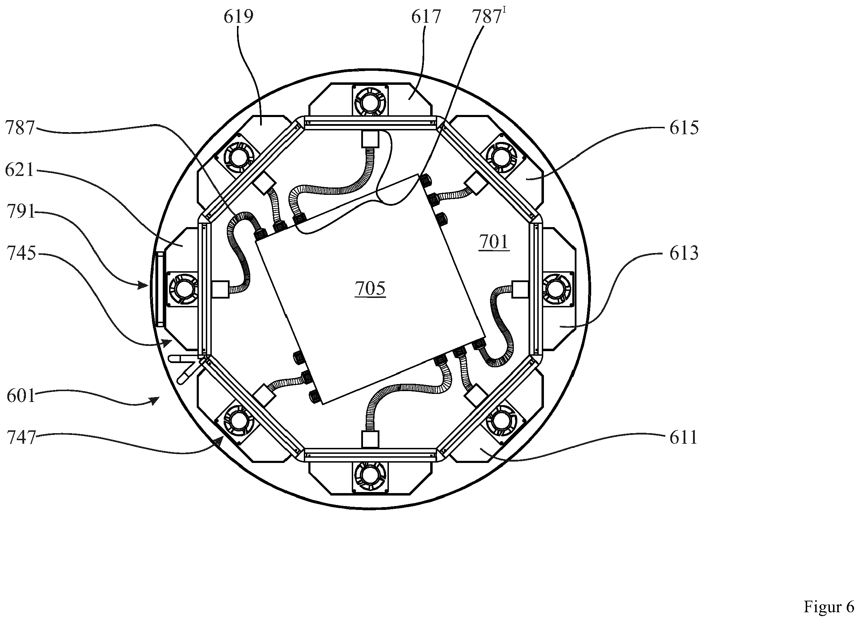

Eine weitere mechanisch stabile Ausführungsform einer Lichttherapiekammer 601 (von oben dargestellt) ist in Figur 6 zu sehen. Die als Teil des Deckels 701 mit diesem mechanisch fest verbundene Elektronikbox 705, die eine wasserdichte Elektronikbox 705 ist, ist über in Leerrohren geführte elektronische Verbindungen 787, 787^{I} an die Module 611, 613, 615, 617, 619, 621 angeschlossen. Die ortsfesten Module 611, 613, 615, 617, 619 können mechanisch fixiert an den elektronischen Verbindungen 787, 787^{I} angeschlossen sein. Die Türflügel 745, 747 sind mit Kontaktflächen ausgestattet, sodass an dem Deckel 701 anliegende Türflügel 745, 757 eine Verbindung über in Leerrohre geführte Kabel, die die elektronische Verbindung 787 schaffen, herstellen können. Das Bedienfeld 791 ist mit einem integrierten Energiespeicher ausgestattet, sodass das Display des Bedienfelds 791 auch leuchtet, wenn der das Bedienfeld 791 tragende Türflügel 745 in der geöffneten Position stehen sollte. Sind berührungslose Kontakte Teile der elektronischen Verbindung 787, so kann ein Informationsaustausch zwischen Elektronikbox 705 und dem Bedienfeld 791, das auf dem Türflügel 745 sitzt, stattfinden, obwohl der Türflügel 745 keinen Kontakt mehr mit dem Deckel 701 hat.

Figur 7 verdeutlicht, dass vorteilhafterweise die Lichttherapiekammer 801 nicht nur eine erste Einheit 921 für Steuerungs- und Überwachungsaufgaben hat, sondern eine zweite Einheit 993 für Steuerungs- und Überwachungsaufgaben, die irgendwo als Teil eines Cloud-Zentralrechners 995 angesiedelt ist, ebenfalls Einfluss auf die Lichttherapiekammer 801 nehmen kann. Die Lichttherapiekammer 801 ist über eine Internetverbindung 989, 989^{I} mit einem Zentralrechner 995 verbunden. Über eine ähnliche Internetverbindung 989^{I} kann eine zweite Steuereinheit 993 Einstellungen für die Lichttherapiekammer 801 an dem Zentralrechner 995 vornehmen. In dem Zentralrechner 995 werden Daten von mehreren Lichttherapiekammern wie der Lichttherapiekammer 801 zusammengeführt, um auf einer größeren, im Speicher des Zentralrechners 995 abgelegten Datenbasis das Verhalten von Lichttherapiekammern 1, 401, 601, 801 ähnlichen Typs zu ermitteln und Lebensdauern, Wartungszyklen und Reparaturmaßnahmen vorherzusehen, zumindest aber rechtzeitig ergreifen zu können. So kann u. a. von dem Zentralrechner 995 auch mitgeschrieben werden, wie häufig die Türflügel 945, 947 geöffnet worden sind, d. h., wie häufig Personen vorgehabt haben, die Lichttherapiekammer 801 durch die Tür 943 zu betreten. Diese Daten, die Daten zu der Anzahl der Türöffnungen, können mit den Daten, wie häufig die Lichtquellen (siehe z. B. die Lichtquellen 53, 53^{I}, 53^{II}, 53^{III} nach Figur 1, siehe z. B. die Leuchtmittel 353, 353^{I}, 353^{II}, 353^{III} nach Figur 3) eingeschaltet worden sind, korreliert werden; die Daten werden vorteilhafterweise miteinander korreliert. In dem Wissen, um welchen Anteil jeder Einschaltvorgang der Lichtquellen 53, 53^{I}, 53^{II}, 53^{III} (nach Figur 1) bzw. der Leuchtmittel 353, 353^{I}, 353^{II}, 353^{III} (nach Figur 3) diese altern lässt, kann zurückgezählt werden, wie häufig noch die Lichttherapiekammer 901 eingeschaltet werden darf, bis womöglich die ersten Lichtquellen ausfallen werden.

In der am höchsten Punkt der Lichttherapiekammer 801 installierten Elektronikbox 905 sind die Schaltungen für die verschiedenen Messungen, Überwachungen, Untersuchungen und Kontrollen der Lichttherapiekammer 801 installiert. Diese Geräte, wie ein Überwachungsschaltkreis 996, ein Zeitmodul 997, eine Spannungsmesseinheit 998, eine Stromsteuerung 999 und ein Homogenitätssensor 1000, bilden die erste Steuereinheit bzw. sind Teil der ersten Steuerungs- und Überwachungseinheit 921.

Der Zentralrechner 995 oder die Steuereinheit 993 des Zentralrechners 995 kann die erste Steuerungs- und Überwachungseinheit 921 dominieren. Der Zentralrechner 995 oder die Steuereinheit 993 des Zentralrechners 995 können Steuerungen und Steuerungsbefehle der ersten Steuerungs- und Überwachungseinheit 921 außer Kraft setzen, also im steuerungstechnischen Sinne "überschreiben". In einer solchen Situation wird der Zentralrechner 995 als Mastergerät bezeichnet. Die erste Steuerungs- und Überwachungseinheit 921 ist das Slavegerät zu dem Mastergerät Zentralrechner 995.

Eine weitere Möglichkeit der Einflussnahme auf die erste Steuerungs- und Überwachungseinheit 921 ist durch das Bedienfeld 991 gegeben. Das Bedienfeld 991 ist auf dem ersten Türflügel angebracht und kann einem Benutzer der Lichttherapiekammer 801 entgegengeschwenkt werden. Außerdem kann das Bedienfeld 991 in seiner Lage bzw. in seiner Winkelneigung angepasst werden, sodass die Lesbarkeit der angezeigten Zeichen und Symbole auf dem Bedienfeld 991 verbessert werden kann.

Stellt ein Benutzer der Lichttherapiekammer 801 ein (Lichttherapie-)Programm ein, dass aufgrund von Daten des Zentralrechners 995 nicht zu starten ist (weil z. B. der Nutzer sich der Gefahr aufgrund seines zuletzt praktizierten Konsums aussetzt, eine Überdosis elektromagnetischer Strahlung zu erhalten), kann der Zentralrechner 995 entgegen der Weisung von dem Bedienfeld 991 der Steuerungs- und Überwachungseinheit 921 vorgeben, dieses Programm nicht auszuführen, sondern z. B. nur ein in der Intensität reduziertes Ersatzprogramm.

Ein allgemeiner bzw. zentraler Überwachungsschaltkreis 996 überwacht die anderen Schaltkreise und Funktionsgruppen und Baugruppen der ersten Steuerungs- und Überwachungseinheit 921.

Zeigt das Zeitmodul 997 an, dass die maximale Betriebszeit für ein bestimmtes Farbprogramm erreicht ist (z. B. sollte in manchen Anwendungsfällen eine Blaulichttherapie maximal 15 Minuten betragen), meldet der Überwachungsschaltkreis 996 über eine seiner Verbindungen, d. h. entweder über das Datenfunkmodul 990 oder über die sonstige Internetverbindung 989 an den Zentralrechner 995, dass die Lichttherapiekammer 801 in einem Pausenmodus ist. Stellt der Zentralrechner 995 fest, dass der Pausenmodus zu lange andauert, kann automatisch ein Service-Techniker oder ein Schulungspersonal informiert und eingebunden werden.

Stellt die Spannungsmesseinheit 998 fest, dass zwar an den elektrischen Kontakten der Türflügel 945, 947 die richtige elektrische Spannung anliegt, wenn diese geschlossen sind, stellt aber der Homogenitätssensor 1000 fest, dass die Lichtverteilung in der Lichttherapiekammer 801 ungleichmäßig ist, so kann auf einen nicht ordnungsgemäß geschlossenen Türflügel 945, 947 zurückgeschlossen werden. Diese Information wird auch an den Zentralrechner 995 über z. B. die Funkmodulschnittstelle 990 weitergeleitet. Eine Person an der zweiten Steuerungs- und Überwachungseinheit 993 kann informiert werden und diese Person kann nach dem Rechten schauen. Auch unterbindet der Zentralrechner 995 die Betriebsaufnahme der Lichttherapiekammer 801 zunächst einmal für das Erste. Die Spannungsmesseinheit 998 misst eine elektrische Spannung anhand eines Spannungsabfalls an einem Vorwiderstand, der als Shunt genutzt wird. Auf diese Weise ist es möglich, auch den Strom durch die Lichtquellen, wie die Lichtquellen 53, 53^{I}, 53", 53^{III} oder durch die Leuchtmittel 353, 353^{I}, 353^{II}, 353^{III} (vgl. Figuren 1, 2 und 3), zu kontrollieren.

Mit Hilfe der Stromsteuerung 999 kann die maximale Helligkeit in der Lichttherapiekammer 801 begrenzt werden. Wird über das Bedienfeld 991 eingestellt, dass ein Kind sich einer Lichttherapie unterziehen soll, sorgt die Stromsteuerung 999 für einen geringeren Strom an die Lichtquellen wie die in der Figur 1 gezeigten Lichtquellen 53, 53^{I}, 53^{II}, 53^{III}.

Die in den einzelnen Figuren gezeigten Ausgestaltungsmöglichkeiten lassen sich auch untereinander in beliebiger Form verbinden.

So ist es u. a. auch möglich, andere oder auch mehrere Bedienfelder 591, 791, 991 an einer Lichttherapiekammer 1, die in den Figuren 1 und 2 gezeigt ist, anzubringen.

Ein günstiger Ort für die Elektronikbox 505, 705, 905 ist das Dach der Lichttherapiekammer. Ist eine Lichttherapiekammer 1, 401, 601, 801 mit weniger Steuerungsfunktionen und einer geringeren Anzahl an Überwachungsschaltkreisen ausgestattet, können diese in dem Bedienfeld 591, 791, 991 integriert sein (z. B. einem Tablet-PC). Das bedeutet, eine gesonderte Elektronikbox 505, 705, 905 kann entfallen, die erste Steuerungs- und Überwachungseinheit 121, 921 und das Bedienfeld 591, 791, 991 können zu einer einzigen Einheit zusammengefasst sein. Vorteilhafterweise gibt es aber trotzdem einen hiervon unabhängigen Zentralrechner 995, der das Mastergerät der gesamten Anlage, ggf. aus mehreren Lichttherapiekammern 1, 401, 601, 801, ist.

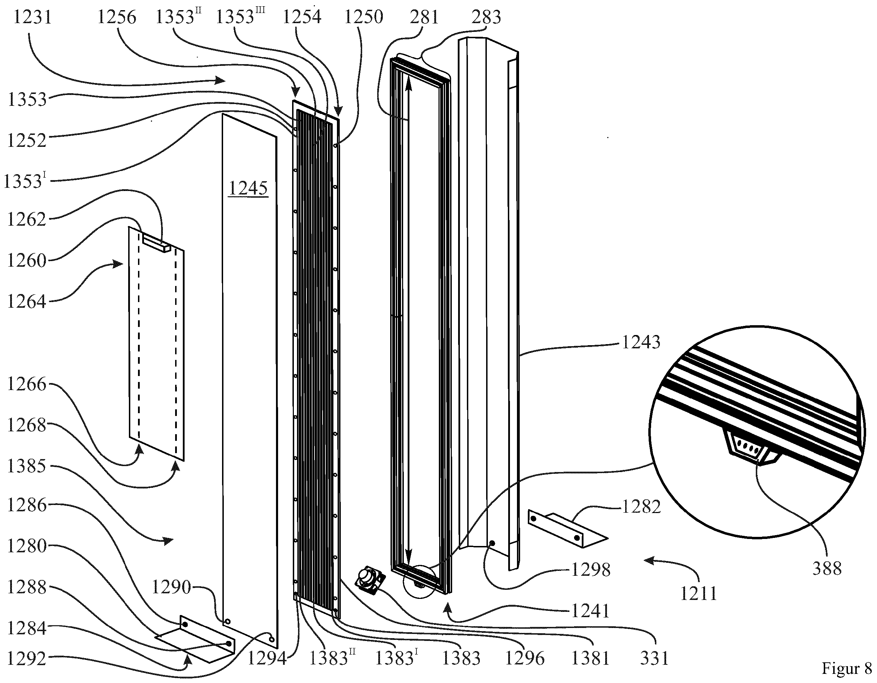

Figur 8 zeigt ein weiteres Beispiel eines Moduls 1211 in Explosionsdarstellung, das, wenn es als eine zusammengebaute Einheit vorliegt, als ein erstes Modul 1211 mit einem weiteren Modul, wie dem Modul 211 in Figur 3, für den Aufbau einer Lichttherapiekammer, wie der Lichttherapiekammer 1 in Figur 1, verbindbar ist. Das Innenleben des Moduls 1211 wird durch ein erstes Panel 1231 gebildet. Zu dem ersten Panel 1231 gehört ein Trägerrahmen 1241 und ein LED-Träger 1381. Wie bei dem Modul 211, das in Figur 3 gezeigt ist, weist der LED-Träger 1381 mehrere Leuchtmittel auf, wie die Leuchtmittel 1353, 1353^{I}, 1353^{II}, 1353^{III}, die in LED-Streifen angeordnet sind, wie den LED-Streifen 1383, 1383^{I}, 1383^{II}. Der Trägerrahmen 1241 stabilisiert und schützt den LED-Träger 1381 an dessen (beiden) Schmalseite(n). Für die Kühlung der Leuchtmittel 1353, 1353^{I}, 1353^{II}, 1353^{III} im Betrieb sorgt ein Ventilator 331. Eine elektrische bzw. elektronische Versorgung erfolgt über einen Stecker 388 an dem Trägerrahmen 1241. Der geerdete Trägerrahmen 1241 kann zusammen mit gleichartigen Trägerrahmen anderer bzw. weiterer Module einer Lichttherapiekammer (vgl. z. B. Lichttherapiekammer 401 in Figur 5) einen Faraday-Käfig bilden. Zusammen mit einer Blende 1243 ist auf diesem Weg sogar möglich, einen mehrschichtigen Schutz einer in der Kammer befindlichen Person, z. B. vor Funkmastenstrahlung, zu bieten. Der LED-Träger 1381 ist an seinen hochseitigen Randbereichen, die einer erste Ebene 1245 zugewandt sind, mit einer ersten Reihe 1254 von Halterungsstellen und einer zweiten Reihe 1256 von Halterungsstellen ausgestattet. Entlang der parallel und beidseitig zu allen LED-Streifen 1383, 1383^{I}, 1383" des Moduls 1211 angeordneten Reihen 1254, 1256 sind jeweils mehrere diskrete Halterungsstellen, wie eine erste Halterungsstelle 1250 und eine zweite Halterungsstelle 1252, vorhanden. Die Halterungsstellen 1250, 1252 sind aus einem in den LED-Träger 1381 eingesetzten magnetischen Material gebildet. Anders gesagt, die Halterungsstellen 1250, 1252 sind kleine Magnete.

In einem weiteren, leicht abgewandelten Ausführungsbeispiel (im Vergleich zu dem Ausführungsbeispiel eines Moduls 1211 nach Figur 8) kann ein äußerer Rahmen des LED-Trägers 1381 aus einem magnetischen, z. B. eisenhaltigen, Material gebildet sein. Eine Halterung für eine Abdeckung auf der Streuscheibe ist durch eine gegenüberliegende Kombination von zwei (dauerhaft) magnetisierten Streifen bzw. Magneten (unter Beachtung der gegensinnigen Nord-Süd Magnetpolung) oder durch Verbindung von einem magnetischen Streifen mit einem (dauerhaft) magnetisierten Streifen durch die Streuscheibe hindurch mittels Magnetkraft ausbildbar.

Die erste Ebene des Lichtbestrahlungsraums 1245 eines Moduls 1211 wird durch eine Abdeckung der LEDs mit einer reflektierenden Streuscheibe 1385 gebildet. Die Streuscheibe 1385 ist für Licht transparent. Die Streuscheibe 1385 dient dazu, zusammen mit einer Aluminium-Blende 1243 das Panel 1231, insbesondere den LED-Träger 1381 und den Trägerrahmen 1241, einzuschließen. Die Blende 1243 bildet durch deren U-profilartige Gestalt mit einer flachen Rückwand den stabilisierenden Rücken des Moduls 1211. In einem bodennahen Bereich der Streuscheibe 1385 ist eine erste Standhilfe 1280 auf die Streuscheibe 1385 aufsetzbar. In einem bodennahen Bereich der Blende 1243 ist eine zweite Standhilfe 1282 auf die Blende 1243 aufsetzbar. Die Standhilfen 1280, 1282 dienen dazu, bei einem Aufbau einer Lichttherapiekammer (siehe z. B. die Lichttherapiekammer 1 nach Figur 1) durch einen einzigen Monteur ein erstes Modul 1211 freistehend zu positionieren, um daran ein zweites Modul, wie das zweite Modul 13 in Figur 1, zu befestigen. Die Standhilfen 1280, 1282 weisen eine Standfläche, wie die Standfläche 1284, auf, durch die ein mögliches Umkippen des Moduls 1211, z. B. bereits durch einen leichten Luftzug, weitgehend verhindert wird. Die Standfläche beträgt in einer vorteilhaften Ausführung etwa 5 % der Fläche, die sich aus Lichtabgabehöhe 281 und Lichtabgabebereite 283 ergibt. An den Standhilfen 1280, 1282, die in Figur 8 gezeigt sind, sind jeweils eine erste Rastierhalterung 1286 und eine zweite Rastierhalterung 1288 vorhanden. Die erste Rastierhalterung 1286 und die zweite Rastierhalterung 1288 der ersten Standhilfe 1280 reichen durch Durchführungen 1290, 1292 in der Streuscheibe 1385 hindurch. Die Standhilfen 1280, 1282 sind in dem LED-Träger 1381 an einer ersten Verankerungsstelle 1294 bzw. einer zweiten Verankerungsstelle 1296 befestigbar. Mit Hilfe der ersten Verankerungsstelle 1294 und der zweiten Verankerungsstelle 1296 wird die Streuscheibe 1385 durch die erste Standhilfe 1280 möglichst wenig belastet.

Eine Rastierhalterung ist eine bolzenartige, durch Rastierung gegen ein Selbstlösen gesicherte Verbindung, wie ein vorzugsweise manuell betätigbares Schnellverschlussystem, z. B. ein Bajonettverschluss, zur Montage einer Standhilfe an einem Modul. Eine tragfähige Komponente des Moduls weist für die Aufnahme der bolzenartigen Verbindung ein Gegenstück, wie eine mutterartiges Gewinde oder wie einen Steg, auf.

In einer weiteren, nicht in Figur 8 dargestellten vorteilhaften Ausführung sind die erste und zweite Verankerungsstelle an dem Trägerrahmen (z. B. einem Trägerrahmen wie dem Trägerrahmen 1241 nach Figur 8) ausgebildet.

Die Rastierhalterungen 1286, 1288 können, z. B. durch eine Drehbewegung, in den Verankerungsstellen 1294, 1296 eingerastet werden. Damit ist die Standhilfe 1280 an dem Modul 1211 spielfrei befestigt. Nach Beendigung eines Aufbaus der Lichttherapiekammer (vgl. Lichttherapiekammer 1 nach Figur 1) können die Standhilfen 1280, 1282 von dem Modul 1211 abgenommen werden, nachdem z. B. eine zum Einrasten gegensinnige Drehbewegung an den Rastierhalterungen 1286, 1288 ausgeführt wurde. Die Durchführungen 1290, 1292 können mit Stöpseln (nicht eingezeichnet) verschlossen werden. Ähnlich wie die erste Standhilfe 1280 ist die zweite Standhilfe 1282 an zwei Verankerungsstellen, wie eine dritte Verankerungsstelle 1298, an der Blende 1243 fixierbar bzw. wieder abnehmbar zu befestigen.

In einer weiteren, nicht in Figur 8 dargestellten vorteilhaften Ausführung dienen die erste und die zweite Verankerungsstelle zur Befestigung des Moduls 1211 an einer Bodenplatte, wie der Bodenplatte 107 in Figur 1, wobei die Bodenplatte eine erste Standhilfe bildet. Die zweite Standhilfe 1282 stützt das Modul 1211 zusätzlich in seiner aufrecht stehenden Stellung. Eine Bodenplatte kann dafür Ausnehmungen aufweisen, in die die Standhilfen eingreifen können. Dadurch ist die Bodenplatte zugleich mit den Modulen verankert. Die Bodenplatte beschwert die Standhilfen, wie z. B. die Standhilfe 1282.

Zu dem Modul 1211, das in Figur8 gezeigt ist, gehört eine Abdeckung 1260. Die Abdeckung 1260 ist als Lichtfilter 1264 zur Lichtintensitätsabschwächung ausgebildet. Die Abdeckung 1260 weist einen Griff 1262 auf. An seitlichen Randbereichen der Abdeckung 1260 sind ein erster Halterungsstreifen 1266 sowie ein zweiter Halterungsstreifen 1268 vorhanden, die jeweils ein magnetisches bzw. magnetisiertes Material, wie einen magnetischen Kunststoff, enthalten. Die Halterungsstreifen 1266, 1268 dienen dazu, in Zusammenwirken mit den Reihen 1254, 1256 von Halterungsstellen des LED-Trägers 1381 die Abdeckung 1260 auf der reflektierenden Streuscheibe 1385 anzubringen. Die Abdeckung 1260 weist eine Breite auf, die etwa gleich der Lichtabgabebreite 283 ist. Die Abdeckung 1260 weist als Abdeckplatte eine Höhe auf, die kleiner als die Lichtabgabehöhe 281 ist. Die Höhe der Abdeckung 1260 beträgt in der in Figur 8 gezeigten Ausführungsform etwa die Hälfte der Lichtabgabehöhe 281.

In einer weiteren, nicht in Figur 8 dargestellten vorteilhaften Ausführung erstreckt sich die Abdeckung über die gesamte Lichtabgabehöhe 281. Besonders vorteilhaft ist die Abdeckung dadurch, dass sie aus einem folienartigen Material besteht. Wie ein Vorhang ist die Abdeckung rolloartig aufwickelbar.

Die Abdeckung 1260 ist als ein Lichtfilter 1264 ein optischer Graufilter, der etwa 90 Prozent der durch die erste Ebene 1245 auf die Abdeckung 1260 einfallenden Lichtstrahlung absorbiert. Der Lichtfilter 1264 weist eine Opazität auf. Somit hat der Lichtfilter 1264 eine Lichttransmission von etwa 10 %. Damit kann ein Monteur Funktionstests, z. B. eine Überprüfung der Funktion der Leuchtmittel 1353, 1353^{I}, 1353^{II}, 1353^{III}, vornehmen, ohne geblendet zu werden. Durch die magnetische Halterung der Abdeckung 1260 an dem Modul 1211 hat der Monteur die Hände frei, um gleichzeitig elektronische Messungen an dem Modul 1211 durchführen zu können.

Wird ein Abdeckung 1260 für den Betrieb einer Lichttherapiekammer (siehe z. B. die Kammer 601) in der Kammer weiterhin verwahrt, kann der Lichtfilter 1264 außerdem, wenn dieser bei Benutzung der Lichttherapiekammer eingesetzt wird, mithilfe des Griffs 1262 so positioniert bzw. verschoben werden, dass zuvor ausreichend mit Licht behandelte Körperseiten einer geringeren Lichtintensität ausgesetzt werden, während andere Körperteile noch weiter der Lichteexposition unterzogen werden.

### Bezugszeichenliste

- 1, 401, 601, 801: Lichttherapiekammer
- 3, 403: Lichtbestrahlungsraum
- 5, 405: Zentralbereich des Lichtbestrahlungsraums
- 7: Lichtbestrahlungsraumachse
- 9: Innere
- 11, 211, 611, 1211: erstes Modul
- 13, 613: zweites Modul
- 15, 615: drittes Modul
- 17, 617: viertes Modul
- 19, 619: fünftes Modul
- 21, 421, 621: sechstes Modul
- 23, 423: siebtes Modul
- 25: achtes Modul
- 31, 231, 1231: erstes Panel
- 33: zweites Panel
- 35: Panel, insbesondere weiteres Panel
- 41, 241, 1241: Trägerrahmen
- 43, 43^{I}, 43^{II}, 43^{III}, 43^{IV},: Blende, insbesondere Aluminium-Blende
- 43^{V}, 243, 1243 45, 245, 1245: erste Ebene des Lichtbestrahlungsraums, insbesondere Abdeckebene vor dem LED-Träger bzw. vor den LEDs eines Panels
- 47: zweite Ebene des Lichtbestrahlungsraums
- 49, 49^{I}: dritte Ebene des Lichtbestrahlungsraums
- 51: Lichtabstrahlfläche, insbesondere aus mehreren Bestrahlungsgroßflächen gebildet
- 53, 53^{I}, 53^{II}, 53^{III}: Lichtquelle
- 353, 353^{I}, 353^{II}, 353^{III},: Leuchtmittel
- 1353, 1353^{I}, 1353^{II}, 1353^{III} 61: Bestrahlungsgroßfläche, insbesondere auf der Innenseite des ersten Moduls
- 63: Bestrahlungsgroßfläche, insbesondere auf der Innenseite des zweiten Moduls
- 65: Bestrahlungsgroßfläche, insbesondere auf der Innenseite des dritten Moduls
- 67: Bestrahlungsgroßfläche, insbesondere auf der Innenseite des vierten Moduls
- 69: Bestrahlungsgroßfläche, insbesondere auf der Innenseite des fünften Moduls
- 71: Bestrahlungsgroßfläche, insbesondere auf der Innenseite des sechsten Moduls
- 73: Bestrahlungsgroßfläche, insbesondere auf der Innenseite des siebten Moduls
- 75: Bestrahlungsgroßfläche, insbesondere auf der Innenseite des achten Moduls
- 77: erste Schicht eines Moduls
- 79: zweite Schicht eines Moduls
- 81, 281: Lichtabgabehöhe
- 83, 283: Lichtabgabebreite
- 85: Strahlrichtung
- 87: schmälere Seite eines Moduls
- 89: breitere Seite eines Moduls
- 91: Innenseite
- 93: Außenseite
- 95: Außenlicht abhaltende Hülle
- 97: Außenlicht
- 499: Stuhl
- 101, 501, 701: Deckel
- 103: Lichtabstrahlungshimmel
- 105, 505, 705, 905: Elektronikbox
- 107,507: Bodenplatte
- 109: Fundamentplatte
- 111: Lichtabstrahlungsbodenplatte
- 113: Vieleck, insbesondere Achteck
- 115, 115^{I}, 115^{II}: erstes Standardprofil, insbesondere Vierkantprofil
- 117, 117': zweites Standardprofil, insbesondere Vierkantprofil
- 119: Stellwinkel
- 121, 921: erste Einheit, insbesondere Steuerungs- und Überwachungseinheit
- 131, 331: erster Lüfter, insbesondere Ventilator
- 133: zweiter Lüfter, insbesondere Ventilator
- 135: dritter Lüfter, insbesondere Ventilator
- 541: Tür, insbesondere offene Tür

- 143, 543, 943: Tür, insbesondere geschlossene Tür
- 145, 545, 745, 945: erster Türflügel
- 147, 547, 747, 947: zweiter Türflügel
- 149, 549: erster Türgriff
- 151, 551: zweiter Türgriff
- 171: geschlossene Zelle
- 381, 1381: LED-Träger
- 383, 383^{I}, 383^{II}, 1383,: LED-Streifen bzw. LED-Band
- 1383^{I}, 1383^{II} 385, 1385: reflektierende Streuscheibe
- 587, 587^{I}, 587^{II}, 587^{III},: elektronische Verbindung, insbesondere zu den Lichtquellen
- 787, 787^{I} 388, 588, 588^{I}: Stecker
- 989, 989^{I}: Internetverbindung
- 990: Datenfunkmodul (insbesondere LTE-Schnittstelle)
- 591, 791, 991: Bedienfeld
- 993: Steuereinheit bzw. zweite Steuerungs- und Überwachungseinheit
- 995: Zentralrechner mit Speicher, insbesondere Cloud-Rechner mit Cloud-Speicher
- 996: Überwachungsschaltkreis
- 997: Zeitmodul
- 998: Spannungsmesseinheit
- 999: Stromsteuerung
- 1000: Homogenitätssensor, insbesondere als Baugruppe einer Helligkeitsmesseinheit
- 1250: erste Halterungsstelle, insbesondere Magnet oder magnetisches Material
- 1252: zweite Halterungsstelle, insbesondere Magnet oder magnetisches Material
- 1254: erste Reihe von Halterungsstellen
- 1256: zweite Reihe von Halterungsstellen
- 1260: Abdeckung, insbesondere Abdeckplatte
- 1262: Griff
- 1264: Lichtfilter, insbesondere lichtintensitätsabschwächender Graufilter
- 1266: erster Halterungsstreifen, insbesondere Streifen aus einem Magneten oder Streifen, der ein magnetisches bzw. magnetisiertes Material aufweist
- 1268: zweiter Halterungsstreifen, insbesondere Streifen aus einem Magneten oder Streifen, der ein magnetisches bzw. magnetisiertes Material aufweist
- 1280: erste Standhilfe
- 1282: zweite Standhilfe
- 1284: Standfläche
- 1286: erste Rastierhalterung, insbesondere erste Bajonettschraube
- 1288: zweite Rastierhaltererung, insbesondere zweite Bajonettschraube
- 1290: erste Durchführung
- 1292: zweite Durchführung
- 1294: erste Verankerungsstelle
- 1296: zweite Verankerungsstelle
- 1298: dritte Verankerungsstelle

## Patentansprüche

1. Lichttherapiekammer (1, 401, 601, 801)
für eine Bestrahlung eines Lebewesens, wie einem Menschen, mit elektromagnetischen Wellen im sichtbaren Wellenspektrum,
insbesondere eine mit einer volumenmäßig für eine Aufnahme eines Menschen dimensionierten, z. B. wenigstens zwei Kubikmeter umfassenden, verschließbaren Zelle (171),
mit einer ersten elektronischen Steuerungs- und Überwachungseinheit (121, 921),
die mit mehreren Lichtquellen (53, 53^{I}, 53^{II}, 53^{III} ) der Lichttherapiekammer (1, 401, 601, 801),
wie LEDs, die vorzugsweise zu LED-Streifen (383, 383^{I}, 383^{II}, 1383, 1383^{I}, 1383^{II}) zusammengeschlossen sind,
in einer elektronischen Verbindung (387, 587, 587^{I}, 587^{II}, 787, 787^{I}),
z. B. zum Ein- und Ausschalten und/oder z. B. zur Funktionsüberwachung,
steht,
**dadurch gekennzeichnet, dass**
die Lichttherapiekammer (1, 401, 601, 801) mit einer über eine Internetverbindung (989, 989^{I}, 990),
z. B. die als mobile Internetverbindung ausgestaltet ist,
anzuschließenden zweiten Steuereinheit (993),
die Archivierungsaufgaben (995) wahrnehmen kann,
verbindbar ist,
wobei letztere Steuereinheit (993) einen über die Internetverbindung (989, 989^{I}, 990) herstellbaren Steuerungseingriff in den Betrieb der Lichttherapiekammer (1, 401, 601, 801) aufgrund von Messdaten der ersten Steuerungs- und Überwachungseinheit (121, 921) besitzt,
wobei vorzugsweise die Messdaten eine Funktionstüchtigkeit der Lichttherapiekammer (1, 401, 601, 801) oder einen bevorstehenden Ausfall der Lichttherapiekammer (1, 401, 601, 801) widerspiegeln.

2. Lichttherapiekammer (1, 401, 601, 801) nach Anspruch 1,
**dadurch gekennzeichnet, dass**
die zweite Steuereinheit (993) in Bezug auf den Betrieb der Lichttherapiekammer (1, 401, 601, 801) ein Mastergerät ist,
das eine Schnittstelle für ein Hindern der ersten Steuerungs- und Überwachungseinheit (121, 921),
die als Slavegerät in Abhängigkeit von dem Mastergerät betrieben wird,
an dem Betrieb der Lichttherapiekammer (1, 401, 601, 801) umfasst.

3. Lichttherapiekammer (1, 401, 601, 801) nach einem der vorhergehenden Ansprüche, **dadurch gekennzeichnet, dass**
die Lichttherapiekammer (1, 401, 601, 801) ein Zeitmodul (997) umfasst,
das für ein Messen, ein Speichern und/oder ein Archivieren von Daten (995) zu Betriebszeiten mehrerer gleichartig betriebener Leuchtmittel (353, 353^{I}, 353^{II}, 353^{III}, 1353, 1353^{I}, 1353^{II}, 1353^{III}),
insbesondere von RGB-LEDs,
gestaltet ist,
wobei die Daten, insbesondere über die Internetverbindung (989, 989^{I}, 990), der zweiten Steuereinheit (993) zur Verfügung gestellt werden.

4. Lichttherapiekammer (1, 401, 601, 801) nach einem der vorhergehenden Ansprüche, **dadurch gekennzeichnet, dass**
die zweite Steuereinheit (993) Teil eines disloziert bzw. entfernt angeordneten Zentralrechners (995) ist,
der sich vorzugsweise in einer Cloud befindet und/oder ein Gerät für eine Fernsteuerung der Lichttherapiekammer (1, 401, 601, 801) darstellt.

5. Lichttherapiekammer (1, 401, 601, 801) nach einem der vorhergehenden Ansprüche, **dadurch gekennzeichnet, dass**
die erste Steuerungs- und Überwachungseinheit (121, 921) wenigstens einen Überwachungsschaltkreis (996) umfasst,
der eine Funktionstüchtigkeit der Lichtquellen (53, 53^{I}, 53^{II}, 53^{III}), ihrer Leuchtmittel (353, 353^{I}, 353^{II}, 353^{III}, 1353, 1353^{I}, 1353^{II}, 1353^{III}) oder einzelner davon und/oder der Lichttherapiekammer (1, 401, 601, 801) kontrolliert.

6. Lichttherapiekammer (1, 401, 601, 801) nach Anspruch 5,
**dadurch gekennzeichnet, dass**
der Überwachungsschaltkreis (996) für eine Betriebsstrommessung aller Leuchtmittel (353, 353^{I}, 353^{II}, 353^{III}, 1353, 1353^{I}, 1353^{II}, 1353^{III}) gestaltet ist und ein Fehlersignalgenerator vorhanden ist, der bei Unterschreitung oder Überschreitung eines erwarteten Betriebsstroms ein Signal an die erste Steuerungs- und Überwachungseinheit (121, 921) und/oder zweite Steuereinheit (993) sendet.

7. Lichttherapiekammer (1, 401, 601, 801) nach Anspruch 5 oder nach Anspruch 6,
**dadurch gekennzeichnet, dass**
der Überwachungsschaltkreis (996) eine kumulierte Betriebszeit pro zuvor betriebenem LED-Farbton misst,
um vorzugsweise ein Datum mit einer Gesamtbetriebszeit, aufgeschlüsselt nach einzelnen Farbtönen, vorzuhalten,
wobei insbesondere mehrere Farbtöne auswählbar sind (591, 791, 991), die im sichtbaren Wellenlängenbereich angesiedelt sind.

8. Lichttherapiekammer (1, 401, 601, 801) nach einem der vorhergehenden Ansprüche, **dadurch gekennzeichnet, dass**
mehrere Lichtquellen (53, 53^{I}, 53^{II}, 53^{III}) oder mehrere Leuchtmittel (353, 353^{I}, 353^{II}, 353^{III}, 1353, 1353^{I}, 1353^{II}, 1353^{III}) in einer Reihenschaltung, z. B. in einer auf einem Streifen (383, 383^{I}, 383^{II}) angeordneten LED-Reihenschaltung, betrieben werden,
zu denen gleichartig viele Lichtquellen (53, 53^{I}, 53^{II}, 53^{III}) oder gleichartig viele Leuchtmittel (353, 353^{I}, 353^{II}, 353^{III}, 1353, 1353^{I}, 1353^{II}, 1353^{III}), z.B. ein zweiter Streifen, in einer parallel verschalteten zweiten Reihenschaltung angeordnet sind.

9. Lichttherapiekammer (1, 401, 601, 801) nach einem der vorhergehenden Ansprüche, **dadurch gekennzeichnet, dass**
die Lichtquellen (53, 53^{I}, 53^{II}, 53^{III}), insbesondere ihre Leuchtmittel (353, 353^{I}, 353^{II}, 353^{III}, 1353, 1353^{I}, 1353^{II}, 1353^{III}), in der Lichttherapiekammer (1) durch eine Stromsteuerung mit elektrischem Strom versorgt werden,
wobei vorzugsweise die Stromsteuerung (999) verschiedene elektrische Ströme für verschiedene Lichtquellen (53, 53^{I}, 53^{II}, 53^{III}), insbesondere für ihre Leuchtmittel (353, 353^{I}, 353^{II}, 353^{III}, 1353, 1353^{I}, 1353^{II}, 1353^{III}), einstellen kann.

10. Lichttherapiekammer (1, 401, 601, 801) nach einem der vorhergehenden Ansprüche, **dadurch gekennzeichnet, dass**
die erste Steuerungs- und Überwachungseinheit (121, 921) eine Spannungsmesseinheit (998) umfasst, die an wenigstens einem Vorwiderstand, z. B. einem Widerstand vor mehreren LEDs, die vorzugsweise auf einem gemeinsamen Träger angeordnet sind, eine Funktionstüchtigkeit der von ihr überwachten Lichtquellen (53, 53^{I}, 53^{II}, 53^{III}) oder Leuchtmittel (353, 353^{I}, 353^{II}, 353^{III}, 1353, 1353^{I}, 1353^{II}, 1353^{III}) überwacht.

11. Lichttherapiekammer (1, 401, 601, 801) nach einem der vorhergehenden Ansprüche, **dadurch gekennzeichnet, dass**
die Lichttherapiekammer (1, 401, 601, 801) wenigstens einen Sensor (1000) umfasst, der eine Homogenität einer Lichtfläche im Inneren der Lichttherapiekammer (1, 401, 601, 801) überwacht.

12. Lichttherapiekammer (1, 401, 601, 801) nach einem der vorhergehenden Ansprüche, **dadurch gekennzeichnet, dass**
die erste Steuerungs- und Überwachungseinheit (121, 921) eine Einheit, insbesondere mit wenigstens einem Sensor (1000), für eine Helligkeitsmessung, insbesondere für eine punktbezogene Helligkeitsmessung, umfasst und eine Auswerteeinheit, die bei einer Abweichung eines Helligkeitswerts von einem vorbestimmten Helligkeitswert weitere Untersuchungen zu einer Funktionstüchtigkeit der Lichtquellen (53, 53^{I}, 53^{II}, 53^{III}), insbesondere ihre Leuchtmittel (353, 353^{I}, 353^{II}, 353^{III}, 1353, 1353^{I}, 1353^{II}, 1353^{III}), der Lichttherapiekammer (1, 401, 601, 801) durchführt.

13. Lichttherapiekammer (1, 401, 601, 801) nach einem der vorhergehenden Ansprüche, **dadurch gekennzeichnet, dass**
die erste Steuerungs- und Überwachungseinheit (121, 921) zumindest eine der folgenden Überwachungsfunktionen, vorzugsweise mehrere der folgenden Überwachungsfunktionen, wahrnehmen kann:
eine Helligkeitsmessung,
eine Helligkeitsprüfung der Lichtquellen (53, 53^{I}, 53^{II}, 53^{III}) innerhalb eines Helligkeitsmessfensters,
eine Gesamtstrommessung,
einen Betriebsstundenzähler,
eine Lüfterüberwachung oder mehrere Lüfterüberwachungen,
eine Personenpräsenzüberwachung,
eine Personenaktivitätsüberwachung,
eine Personenherzschlagsüberwachung,
eine Personenlebendüberwachung,
eine Alterskompensation, insbesondere der Leuchtmittel (353, 353^{I}, 353^{II}, 353^{III}, 1353, 1353^{I}, 1353^{II}, 1353^{III}),
eine Türpositionsprüfung, insbesondere anhand von einem oder mehreren Mikroschaltern.

14. Verfahren zur Überwachung einer Lichttherapiekammer (1, 401, 601, 801), insbesondere zur Überwachung einer Lichttherapiekammer (1, 401, 601, 801) nach einem der vorhergehenden Ansprüche,
**dadurch gekennzeichnet, dass**
eine Überwachungseinheit eine Funktionstüchtigkeit von Leuchtmitteln (353, 353^{I}, 353^{II}, 353^{III}, 1353, 1353^{I}, 1353^{II}, 1353^{III}) der Lichttherapiekammer (1, 401, 601, 801) überwacht und bei Ausfall, Verschlechterung und/oder einem unerwünschten Betriebszustand von Leuchtmitteln (353, 353^{I}, 353^{II}, 353^{III}, 1353, 1353^{I}, 1353^{II}, 1353^{III}) einen Wartungs-, Reparatur- und/oder Besichtigungsauftrag an einen Zentralrechner (995) übermittelt.

15. Verfahren zur Überwachung einer Lichttherapiekammer (1, 401, 601, 801) nach Anspruch 14,
**dadurch gekennzeichnet, dass**
die Überwachungseinheit eine Berechnung einer prognostizierenden Restbetriebszeit für Leuchtmittel (353, 353^{I}, 353^{II}, 353^{III}, 1353, 1353^{I}, 1353^{II}, 1353^{III}) unternimmt und nach Kriterien einer zweiten Steuereinheit (993) einen Betrieb der Lichttherapiekammer (1, 401, 601, 801) unterbindet.
